(19) Europäisches Patentamt
European Patent Office
Office européen des brevets

(11) Veröffentlichungsnummer: **0 072 347**
**B1**

(12) # EUROPÄISCHE PATENTSCHRIFT

(45) Veröffentlichungstag der Patentschrift: **13.11.85**

(51) Int. Cl.[4]: **C 07 D 239/46**, C 07 D 239/52, C 07 D 251/16, C 07 D 251/42, C 07 D 251/46, A 01 N 47/36

(21) Anmeldenummer: **82810323.4**

(22) Anmeldetag: **02.08.82**

(54) **N-Phenylsulfonyl-N'-pyrimidinyl- und -triazinylharnstoffe.**

(30) Priorität: **06.08.81 CH 5075/81**
**08.04.82 CH 2205/82**

(43) Veröffentlichungstag der Anmeldung: **16.02.83 Patentblatt 83/7**

(45) Bekanntmachung des Hinweises auf die Patenterteilung: **13.11.85 Patentblatt 85/46**

(84) Benannte Vertragsstaaten: **AT BE CH DE FR GB IT LI NL SE**

(56) Entgegenhaltungen:
**EP - A - 0 009 419**
**EP - A - 0 023 140**
**EP - A - 0 023 422**
**EP - A - 0 044 807**
**EP - A - 0 044 808**
**EP - A - 0 044 809**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Meyer, Willy, Talweg 49, CH-4125 Riehen (CH)**
Erfinder: **Föry, Werner, Dr., Benkenstrasse 65, CH-4054 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die vorliegende Erfindung betrifft neue, herbizid wirksame und pflanzenwuchsregulierende N-Phenylsulfonyl-N'-pyrimidinyl- und -triazinylharnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums.

Die erfindungsgemäßen N-Phenylsulfonyl-N'-pyrimidinyl- und -triazinylharnstoffe entsprechen der allgemeinen Formel I

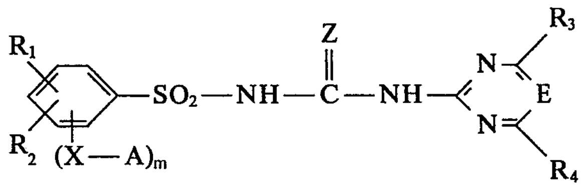

(I)

worin

A einen durch Halogen, $C_1$—$C_4$-Alkoxy, $C_1$—$C_4$-Alkylthio, $C_1$—$C_4$-Alkylsulfinyl, $C_1$—$C_4$-Alkylsulfonyl, $C_1$—$C_4$-Halogenalkoxy, $C_1$—$C_4$-Halogenalkylthio, $C_1$—$C_4$-Halogenalkylsulfinyl oder $C_1$—$C_4$-Halogenalkylsulfonyl substituierten $C_1$—$C_6$-Alkylrest oder einen durch die aufgezählten Reste substituierten $C_2$—$C_6$-Alkenylrest,
E die Methingruppe oder Stickstoff,
X Sauerstoff, Schwefel, eine Sulfinyl- oder Sulfonylbrücke,
Z Sauerstoff oder Schwefel,
m die Zahl eins oder zwei,
$R_1$ Wasserstoff, Halogen, $C_1$—$C_5$-Alkyl, $C_2$—$C_5$-Alkenyl oder einen Rest —Y—$R_5$,
$R_2$ Wasserstoff, Halogen, $C_1$—$C_5$-Alkyl, $C_2$—$C_5$-Alkenyl, $C_1$—$C_4$-Halogenalkyl, oder einen Rest —Y—$R_5$, —$COOR_6$, —$NO_2$ oder —CO—$NR_7$—$R_8$,
$R_3$ Wasserstoff, $C_1$—$C_4$-Alkyl, $C_1$—$C_4$-Alkoxy, $C_1$—$C_4$-Alkylthio, $C_1$—$C_4$-Halogenalkyl, $C_1$—$C_4$-Halogenalkoxy, Halogen oder Alkoxyalkyl mit höchstens 4 Kohlenstoffatomen,
$R_4$ $C_1$—$C_4$-Halogenalkoxy,
$R_5$ und $R_6$ je $C_1$—$C_5$-Alkyl, $C_2$—$C_5$-Alkenyl oder $C_2$—$C_6$-Alkinyl,
$R_7$ und $R_8$ unabhängig voneinander Wasserstoff, $C_1$—$C_5$-Alkyl, $C_2$—$C_5$-Alkenyl oder $C_2$—$C_6$-Alkinyl und
Y Sauerstoff, Schwefel, eine Sulfinyl- oder Sulfonylbrücke bedeuten,

wobei A auch für den unsubstituierten $C_2$—$C_6$-Alkenylrest stehen kann, wenn gleichzeitig X Sauerstoff bedeutet, sowie den Salzen dieser Verbindungen.

Harnstoffverbindungen, Triazinverbindungen und Pyrimidinverbindungen mit herbizider Wirkung sind allgemein bekannt. Kürzlich wurden Arylsulfamoyl-heterocyclyl-aminocarbamoylverbindungen mit herbizider und pflanzenwuchsregulierender Wirkung, beispielsweise im holländischen Patent 121 788, den europäischen Patentpublikationen Nr. 9 419, 23 422, dem US Patent Nr. 4 127 405, in der DE-OS 2 715 786 oder in der FR-PS 1 468 747 beschrieben.

In den Definitionen ist unter Alkyl geradkettiges oder verzweigtes Alkyl zu verstehen; z. B.: Methyl, Äthyl, n-Propyl, i-Propyl, die vier isomeren Butyl, n-Amyl, 2-Amyl, 3-Amyl, n-Hexyl oder i-Hexyl.

Unter Alkoxy ist zu verstehen: Methoxy, Äthoxy, n-Propyloxy, i-Propyloxy und die vier isomeren Butyloxyreste, insbesondere aber Methoxy, Äthoxy oder i-Propoxy.

Beispiele für Alkylthio sind Methylthio, Äthylthio, n-Propylthio, i-Propylthio und n-Butylthio, insbesondere aber Methylthio und Äthylthio.

Beispiele für Alkenylreste sind Vinyl, Allyl, Isopropenyl, 1-Propenyl, 1-Butenyl, 2-Butenyl, 3-Butenyl, 1-Isobutenyl, 2-Isobutenyl, 1-Pentenyl, 2-Pentenyl, 3-Pentenyl und 4-Pentenyl, insbesondere aber Vinyl, Allyl und 4-Pentenyl.

Beispiele für Alkylsulfinyl sind Methylsulfinyl, Äthylsulfinyl, n-Propylsulfinyl und n-Butylsulfinyl, insbesondere aber Methylsulfinyl und Äthylsulfinyl.

Beispiele für Alkylsulfonyl sind Methylsulfonyl, Äthylsulfonyl, n-Propylsulfonyl und n-Butylsulfonyl, insbesondere aber Methylsulfonyl und Äthylsulfonyl.

Unter Halogen in den Definitionen sowie in Halogenalkyl, -alkoxy, -alkylsulfinyl, -alkylsulfonyl und -alkylthio sind Fluor, Chlor und Brom, vorzugsweise jedoch Fluor und Chlor zu verstehen.

Bevorzugte Halogenalkylreste, die auch Bestandteile größerer Substituentengruppen sein können, wie z. B. eines Halogenalkoxy- oder Halogenalkylthiorestes, sind Difluormethyl, Trifluormethyl, Perfluoräthyl, 2,2,2-Trifluoräthyl, Chlorfluormethyl, Bromfluormethyl, 2-Chloräthyl, 1,1,2,2-Tetrafluoräthyl, 2-Fluoräthyl, 2,2,2-Trichloräthyl, Bromdifluormethyl, Chlordifluormethyl, 1,1,2-Trifluor-2-chloräthyl, 1,1,2-Trifluor-2-bromäthyl, 1,1,2,3,3,3-Hexafluorpropyl, insbesondere aber Difluormethyl, 2,2,2-Trifluoräthyl und Perfluoräthyl.

Alkinylresten in den Definitionen der obigen Symbole entsprechen in der Regel Propargyl, 2-Butinyl, 3-Butinyl, sowie isomere Pentinyl- oder Hexinylreste, vorzugsweise ist der Alkinylrest jedoch durch Propargyl oder 2- oder 3-Butinyl verkörpert.

Die Erfindung umfaßt ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Äthylamin, Propylamin, i-Propylamin, die vier isomere Butylamine, Dimethylamin, Diäthylamin, Diäthanolamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin und i-Chinolin, insbesondere aber Äthyl-, Propyl-, Diäthyl- oder Triäthylamin, vor allem aber iso-Propylamin und Diäthanolamin.

Beispiele für quaternäre Ammoniumbasen sind im allgemeinen die Kationen von Halogenammoniumsalzen, z. B. das Tetramethylammoniumkation, das Trimethylbenzylammoniumkation, das Triäthylbenzylammoniumkation, das Tetraäthylammoniumkation, das Trimethyläthylammoniumkation, aber auch das Ammoniumkation.

Von den erfindungsgemäßen Verbindungen der Formel I sind die Verbindungen bevorzugt, in denen entweder

a) X oder
b) Z Sauerstoff oder
c) m die Zahl eins bedeuten oder
d) die Reste $R_3$ und $R_4$ zusammen nicht mehr als 4 Kohlenstoffatome enthalten.

Durch Kombination der bevorzugten Merkmale ergibt sich folgende weiter bevorzugte Gruppe von Verbindungen der Formel I, in denen X und Z Sauerstoff und m die Zahl eins bedeuten und der Rest —X—A die ortho-Position zur Sulfonylgruppe besetzt.

Innerhalb dieser Gruppe sind weiterhin die Verbindungen bevorzugt, in denen die Reste $R_3$ und $R_4$ zusammen nicht mehr als 4 Kohlenstoffatome enthalten.

Besonders bevorzugt sind innerhalb dieser Untergruppe die Verbindungen, in denen $R_4$ für Halogenäthoxy und Halogenmethoxy steht. Hiervon sind wiederum die Verbindungen bevorzugt, in denen $R_4$ den 2,2,2-Trifluoräthoxyrest oder den Difluormethoxyrest bedeutet.

Als bevorzugte Einzelverbindungen sind zu nennen:

N-(2-Difluormethoxyphenyl-sulfonyl)-N'-[4-methoxy-6-(2,2,2-trifluoräthoxy)-1,3,5-triazin-2-yl]-harnstoff,
N-[2-(2-Chloräthoxy)-phenylsulfonyl]-N'-[4-methoxy-6-(2,2,2-trifluoräthoxy)-1,3,5-triazin-2-yl]-harnstoff,
N-(2-Trifluormethoxyphenyl-sulfonyl)-N'-(4-difluormethoxy-6-methyl-pyrimidin-2-yl)-harnstoff und
N-(2-Difluormethoxyphenyl-sulfonyl)-N'-(4-difluormethoxy-6-methyl-pyrimidin-2-yl)-harnstoff.

Die Herstellung der Verbindungen der Formel I erfolgt in einem inerten, organischen Lösungsmittel.

Nach einem ersten Verfahren werden die Verbindungen der Formel I erhalten, indem man ein Phenylsulfonamid der Formel II

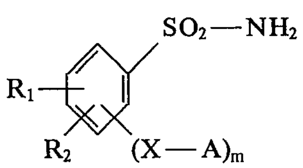

(II)

worin A, $R_1$, $R_2$, X und m die unter Formel I gegebene Bedeutung haben, in Gegenwart einer Base mit einem N-Pyrimidinyl- oder -Triazinylcarbamat der Formel III

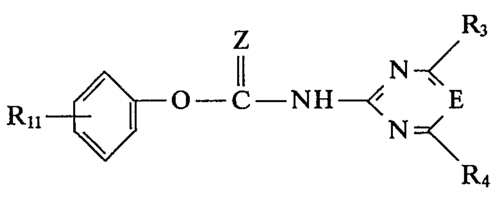

(III)

worin E, $R_3$, $R_4$ und Z die unter Formel I gegebene Bedeutung haben, und $R_{11}$ für Wasserstoff, Halogen, Nitro oder $C_1$—$C_3$-Alkyl steht, umsetzt.

Nach einem zweiten Verfahren gelangt man zu Verbindungen der Formel I, indem man ein Phenylsulfonylisocyanat oder -isothiocyanat der Formel IV

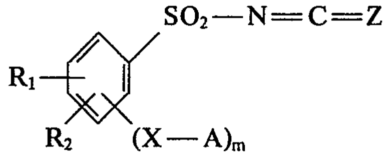

(IV)

worin A, $R_1$, $R_2$, m, X und Z die unter Formel I gegebene Bedeutung haben, gegebenenfalls in Gegenwart einer Base, mit einem Amin der Formel V

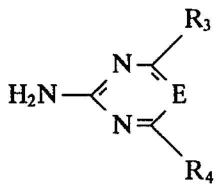

(V)

worin E, $R_3$ und $R_4$ die unter Formel I gegebene Bedeutung haben, umsetzt.

Nach einem weiteren Verfahren werden die Verbindungen der Formel I hergestellt, indem man ein Sulfonamid der oben angegebenen Formel II gegebenenfalls in Gegenwart einer Base mit einem Isocyanat oder Isothiocyanat der Formel VI

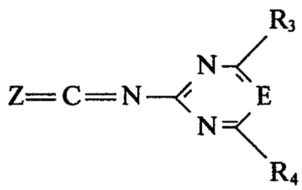

(VI)

worin E, $R_3$, $R_4$ und Z die unter Formel I gegebene Bedeutung haben, umsetzt.

Schließlich kann man die Verbindungen der Formel I auch erhalten, indem man ein N-Phenylsulfonylcarbamat der Formel VII

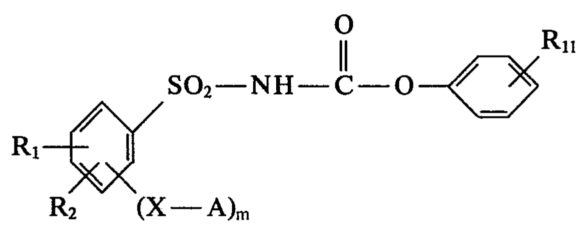

(VII)

worin A, $R_1$, $R_2$, m und X die unter Formel I gegebene Bedeutung haben und $R_{11}$ für Wasserstoff, Halogen, Nitro oder $C_1$—$C_3$-Alkyl steht, mit einem Amin der oben angegebenen Formel V umsetzt.

Die erhaltenen Harnstoffe der Formel I können gewünschtenfalls mittels Aminen, Alkalimetall- oder Erdalkalimetallhydroxiden oder quaternären Ammoniumbasen in Additionssalze übergeführt werden. Dieses geschieht beispielsweise durch Umsetzen mit der äquimolaren Menge Base und Verdampfen des Lösungsmittels.

Die Ausgangsstoffe der Formeln II, IV, VII und ortho-substituierte Hydroxyphenyl- resp. substituierte ortho-Hydroxyphenylsulfonamide der Formel VIII,

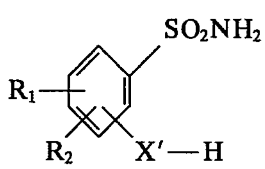

(VIII)

worin $R_1$ und $R_2$ die unter Formel I angegebene Bedeutung haben und X' für Sauerstoff oder Schwefel steht, als Ausgangsprodukte bestimmter Sulfonamid-Vertreter der Formel II, sind aus der europäischen Patentanmeldung 44 807 bekannt.

Die Ausgangsmaterialien der Formeln III, V und VI sind zum Teil bekannt. Neue Verbindungen der Formeln III und VI können nach bekannten Methoden aus entsprechenden Verbindungen der Formel V erhalten werden.

Neue Fluoralkoxy-aminopyrimidine und -triazine der Formel V und ihre Herstellung sowie die Herstellung entsprechender Verbindungen der Formeln III und VI daraus werden in der europäischen Patentanmeldung EP-A 70 804 beschrieben.

Die Umsetzungen zu Verbindungen der Formel I werden vorteilhafterweise in aprotischen, inerten, organischen Lösungsmitteln vorgenommen wie Methylenchlorid, Tetrahydrofuran, Acetonitril, Dioxan, Toluol. Als Basen können sowohl organische Basen wie Amine, wie Triäthylamin, Chinuclidin, Chinolin, Pyridin etc. als auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Reaktionstemperaturen liegen vorzugsweise zwischen −20° und +120°C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt.

Die Endprodukte können durch Einengen und/oder Verdampfen des Lösungsmittels isoliert und durch Umkristallisieren oder Zerreiben des festen Rückstandes in Lösungsmitteln in denen sie sich nicht gut lösen, wie Äther, aromatischen Kohlenwasserstoffen oder chlorierten Kohlenwasserstoffen gereinigt werden.

Die Wirkstoffe der Formel I sind stabile Verbindungen. Ihre Handhabung bedarf keiner vorsorglicher Maßnahmen.

Bei geringeren Aufwandmengen zeichnen sich die Verbindungen der Formel I durch gute selektivwuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d. h. sie werden von der Pflanze aufgenommen und an andere Stellen transportiert, wo sie dann zur Wirkung kommen. So gelingt es beispielsweise, durch Oberflächenbehandlung perenierende Unkräuter bis in die Wurzeln zu schädigen. Die neuen Verbindungen der Formel I wirken bereits bei — im Vergleich zu anderen Herbiziden und Wuchsregulatoren — sehr geringen Aufwandmengen.

Die Verbindungen der Formel I haben außerdem starke pflanzenwuchsregulierende Eigenschaften, die sich in einer Ertragssteigerung von Kulturpflanzen oder Erntegut auswirken kann. Viele Verbindungen der Formel I zeigen darüber hinaus eine konzentrationsabhängige pflanzenwuchshemmende Wirkung. Es werden sowohl Monokotyledonen als auch Dikotyledonen in ihrem Wachstum beeinträchtigt.

So können z. B. die in der Landwirtschaft in tropischen Gegenden häufig als »cover crops« (Bodenbedecker) angepflanzten Leguminosen durch die Verbindungen der Formel I in ihrem Wachstum selektiv gehemmt werden, so daß zwar die Bodenerosion zwischen den Kulturpflanzen verhindert wird, die »cover crops« jedoch nicht zur Konkurrenz für die Kultur werden können.

Eine Hemmung des vegetativen Wachstums erlaubt bei vielen Kulturpflanzen eine dichtere Anpflanzung der Kultur, so daß ein Mehrertrag, bezogen auf die Bodenfläche, erzielt werden kann.

Ein weiterer Mechanismus der Ertragssteigerung mit Wachstumshemmern beruht darauf, daß die Nährstoffe in stärkerem Maße der Blüten- und Fruchtbildung zugute kommen, während das vegetative Wachstum eingeschränkt wird.

Weiter eignen sich die Verbindungen der Formel I um das Keimen von eingelagerten Kartoffeln zu verhindern. Bei Kartoffeln entwickeln sich bei der Einlagerung über den Winter häufig Keime, die Schrumpfen, Gewichtsverlust und Faulen zur Folge haben.

Bei größeren Aufwandmengen werden alle getesteten Pflanzen in ihrer Entwicklung so geschädigt, daß sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung, zur Hemmung des Pflanzenwuchses von monokotylen und dikotylen Pflanzen, insbesondere Gräsern, tropischen Bodenbedeckern und Tabakgeiztrieben und zur Regulierung des Pflanzenwachstums insbesondere von Leguminosen.

Die Verbindungen der Formel I werden in unveränderter Form oder vorzugsweise als Mittel zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Gießen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Frak-

tionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnußöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$—$C_{22}$), wie z. B. die Na- oder K-Salze der Öl- oder Stearinsäure, oder von natürlichen Fettsäuregemischen, die z. B. aus Kokosnuß- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2-Sulfonsäuregruppen und einen Fettsäurerest mit 8—22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z. B. Salze des Phosphorsäureesters eines p-Nonylphenol-(4—14)-Äthylenoxid-Adduktes in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u. a. in folgenden Publikationen beschrieben:

»Mc Cutcheon's Detergents and Emulsifiers Annual« MC Publishing Corp., Ridgewood, New Jersey, 1979.

Sisley and Wood, »Encyclopedia of Surface Active Agents«. Chemical Publishing Co., Inc. New York, 1964.

Die pestiziden Zubereitungen enthalten in der Regel 0,1 bis 95%, insbesondere 0,1 bis 80%, Wirkstoff der Formel I, 1 bis 99,9% eines festen oder flüssigen Zusatzstoffes und 0 bis 25%, insbesondere 0,1 bis 25% eines Tensides.

Insbesondere setzen sich bevorzugte Formulierungen folgendermaßen zusammen (% = Gewichtsprozent):

Emulgierbare Konzentrate

| | |
|---|---|
| Aktiver Wirkstoff: | 1 bis 20%, bevorzugt 5 bis 10% |
| oberflächenaktives Mittel: | 5 bis 30%, vorzugsweise 10 bis 20% |
| flüssiges Trägermittel: | 50 bis 94%, vorzugsweise 70 bis 85%. |

Stäube

| | |
|---|---|
| Aktiver Wirkstoff: | 0,1 bis 10%, vorzugsweise 0,1 bis 1% |
| festes Trägermittel: | 99,9 bis 90%, vorzugsweise 99,9 bis 99%. |

Suspension-Konzentrate

| | |
|---|---|
| Aktiver Wirkstoff: | 5 bis 75%, vorzugsweise 10 bis 50% |
| Wasser: | 94 bis 25%, vorzugsweise 90 bis 30% |
| oberflächenaktives Mittel: | 1 bis 40%, vorzugsweise 2 bis 30%. |

Benetzbare Pulver

| | |
|---|---|
| Aktiver Wirkstoff: | 0,5 bis 90%, vorzugsweise 1 bis 80% |
| oberflächenaktives Mittel: | 0,5 bis 20%, vorzugsweise 1 bis 15% |
| festes Trägermittel: | 5 bis 95%, vorzugsweise 15 bis 90%. |

Granulate

| | |
|---|---|
| Aktiver Wirkstoff: | 0,5 bis 30%, vorzugsweise 3 bis 15% |
| festes Trägermittel: | 99,5 bis 70%, vorzugsweise 97 bis 85%. |

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

## Herstellungsbeispiele

### Beispiel 1

N-(2-Difluormethoxyphenyl-sulfonyl)-N'-[4-methoxy-6-(2,2,2-trifluoräthoxy)-1,3,5-triazin-2-yl]-harnstoff

a) Zur Suspension von 80,3 g 2-Amino-4-chlor-6-methoxy-1,3,5-triazin in 400 ml absolutem Dioxan läßt man in 10 Minuten 124,6 g 2-Difluormethoxyphenyl-sulfonylisocyanat in 100 ml Dioxan zutropfen. Die Reaktionsmischung wird für 3 Stunden auf 90—100° erwärmt. Durch Abkühlen und Einengen der Reaktionslösung erhält man 180 g N-(2-Difluormethoxyphenyl-sulfonyl)-N'-(4-chlor-6-methoxy-1,3,5-triazin-2-yl)-harnstoff, Smp. 167—168°.

b) Eine Mischung aus 8,2 g N-(2-Difluormethoxyphenyl-sulfonyl)-N'-(4-chlor-6-methoxy-1,3,5-triazin-2-yl)-harnstoff, 60 ml Dioxan, 20 ml 2,2,2-Trifluoräthanol und 5,5 g Kaliumcarbonat wird für 12 Stunden bei 55—60° gerührt, dann in 300 ml Wasser aufgenommen und über Aktivkohle filtriert. Nach dem Ansäuern des Gemisches mit 2 n Salzsäure, fällt das Produkt aus und wird durch Filtration abgetrennt. Ausbeute: 7,7 g N-(2-Difluormethoxyphenyl-sulfonyl)-N'-[4-methoxy-6-(2,2,2-trifluoräthoxy)-1,3,5-triazin-2-yl]-harnstoff, Smp. 155—157°.

### Beispiel 2

N-(2-Difluormethoxyphenyl-sulfonyl)-N'-(4-difluormethoxy-6-methyl-pyrimidin-2-yl)-harnstoff

a) In eine Lösung von 62,5 g 2-Amino-4-hydroxy-6-methyl-pyrimidin in 500 ml Wasser, 100 ml 40%iger Natriumhydroxidlösung und 100 ml Dioxan wird über einen Zeitraum von 12 Stunden bei einer Temperatur von 70—75°C gasförmiges Difluorchlormethan eingeleitet. Während dieses Zeitraumes fügt man, jeweils im Abstand von 1 Stunde, in gleichen Portionen insgesamt 160 g festes Natriumhydroxid zu. Durch Abtrennen der organischen Phase, Einengen auf ca. $\frac{1}{10}$ des Volumens, Eingießen des Rückstandes in Wasser und Abtrennen des ausgefallenen Feststoffes erhält man 39,9 g 2-Amino-4-difluormethoxy-6-methyl-pyrimidin, Smp. 136—137°C.

b) Eine Mischung aus 2,5 g 2-Difluormethoxyphenyl-sulfonylisocyanat, 1,75 g 2-Amino-4-difluormethoxy-6-methyl-pyrimidin und 30 ml absolutem Dioxan wird für 2 Stunden bei einer Temperatur von 70—75°C gerührt. Durch Eindampfen der Lösung und Kristallisation des Rückstandes aus Äther erhält man 4,0 g N-(2-Difluormethoxyphenyl-sulfonyl)-N'-(4-difluormethoxy-6-methyl-pyrimidin-2-yl)-harnstoff, Smp. 163—164°C.

### Beispiel 3

N-[2-(2-Äthoxy-äthoxy)-phenyl-sulfonyl]-N'-(4-difluormethoxy-6-methyl-pyrimidin-2-yl)-harnstoff

a) Einer Lösung von 9,0 g (0,05 Mol) 2-Amino-4-difluormethoxy-6-methyl-pyrimidin in 30 ml absolutem Tetrahydrofuran werden nacheinander 3,9 g (0,025 Mol) Chlorameisensäure-phenylester und 0,05 g 4-Dimethylamino-pyridin zugesetzt. Nach Rühren der Lösung bei 20—25°C für 24 Stunden wird das Reaktionsgemisch mit 250 ml Äthylacetat verdünnt und der ausgefallene Niederschlag abgetrennt. Man erhält so 3,1 g 2-Amino-4-difluormethoxy-6-methyl-pyrimidin-hydrochlorid, Smp. 204—205°C (Zers.). Das Filtrat wird zur Entfernung des restlichen Ausgangsstoffes über eine Säule mit Ionenaustauscher I ® (MERCK) eluiert, über Natriumsulfat getrocknet und eingedampft. Man erhält so in Form eines viskosen Öles als Rohprodukt 4,0 g 2-Phenoxycarbonylamino-4-difluormethoxy-6-methyl-pyrimidin, das nach der Kristallisation bei 56—58°C schmilzt.

$^1$H-NMR ($CDCl_3$) : $\delta$ = 2,5 (s, $CH_3$), 6,45 (s, 1H), 7,0—7,5 (5H), 7,56 (t, J = 70 Hz, $CHF_2$) und 9,0 (NH) ppm.

b) Eine Lösung von 3,5 g (0,012 Mol) 2-Phenoxycarbonylamino-4-difluormethoxy-6-methyl-pyrimidin-Rohprodukt und 2,9 g (0,012 Mol) 2-(2-Äthoxy-äthoxy)-benzolsulfonamid in 100 ml absolutem Acetonitril wird mit 1,8 g (0,012 Mol) 1,5-Diazabicyclo(5,4,0)undec-5-en versetzt, für 1 Stunde bei 20—25°C gerührt, dann mit 500 ml Wasser verdünnt und mit Salzsäure angesäuert. Das abgeschiedene Öl wird mit 200 ml Äthylacetat aufgenommen über Natriumsulfat getrocknet und eingedampft. Durch Kristallisieren des öligen Rückstandes aus Diäthyläther und Aceton erhält man als farbloses Kristallisat N-[2-(2-Äthoxy-äthoxy)-phenyl-sulfonyl]-N'-(4-difluormethoxy-6-methyl-pyrimidin-2-yl)-harnstoff, Smp. 90—95°C (Zers.), Ausbeute 2,7 g.

### Beispiel 5

2-Amino-4-chlor-6-difluormethoxy-pyrimidin

Eine Suspension von 16,4 g 2-Amino-4,6-dichlor-pyrimidin in 100 ml 40%iger Natriumhydroxidlösung wird für 1 Stunde bei einer Temperatur von 95—100°C gerührt. Nach Zusatz von 200 ml Dioxan werden bei einer Temperatur von 70—75°C innerhalb von 1 Stunde 20 g gasförmiges Difluorchlormethan eingeleitet. Durch Abtrennen der organischen Phase, Einengen auf ca. $\frac{1}{5}$ des Volumens, Eingießen in Wasser und Abtrennen des festen Niederschlages erhält man 6 g 2-Amino-4-chlor-6-difluormethoxy-pyrimidin, Smp. 118—119°C.

### Beispiel 6

2-Amino-4-difluormethoxy-6-methoxy-pyrimidin

19,2 g 2-Amino-4,6-dimethoxy-pyrimidin-hydrochlorid werden während 2 Stunden auf 150°C erhitzt, wobei sich unter Abspaltung von Methylchlorid 2-Amino-4-hydroxy-6-methoxy-pyrimidin bildet. Nach Zusatz von 80 ml 40%iger Natriumhydroxidlösung und 100 ml Dioxan werden bei 70—75° während $\frac{3}{4}$ Stunden 22 g Difluorchlormethan eingeleitet. Durch Abtrennen der org. Phase, Einengen auf ca. $\frac{1}{5}$ des Volumens, Eingießen in Wasser und Abtrennen des festen Niederschlages erhält man 2,4 g 2-Amino-4-difluormethoxy-6-methoxy-pyrimidin, Smp. 106—107°C.

Beispiel 7

2-Amino-4-methyl-6-[1,1,2-trifluor-2-chlor-äthoxy]-pyrimidin

25 g 2-Amino-4-hydroxy-6-methyl-pyrimidin, 25,6 g Chlortrifluoräthylen, 13,8 g Kaliumhydroxyd und 200 ml Dimethylformamid werden zusammen während 8 Stunden bei 60° im Autoklaven gerührt. Durch Verdünnen mit Wasser, Extrahieren mit Essigester und Eindampfen erhält man ein dunkles Öl. Nach der Zugabe von wenig Methylenchlorid und Abtrennen des gebildeten kristallinen Niederschlages erhält man 8,7 g 2-Amino-4-methyl-6-(1,1,2-trifluor-2-chlor-äthoxy)-pyrimidin, Smp. 73–74°C.

In analoger Weise erhält man die in den anschließenden Tabellen aufgelisteten Endprodukte.

Tabelle I

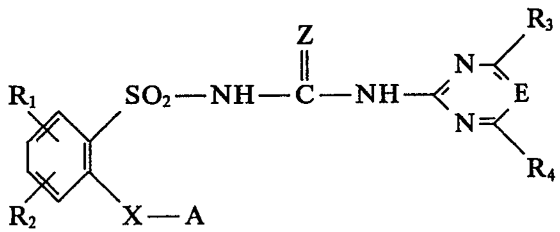

| Nr. | $R_1$ | $R_2$ | X | A | Z | E | $R_3$ | $R_4$ | Smp. [°C] |
|---|---|---|---|---|---|---|---|---|---|
| 1 | H | H | O | $CHF_2$ | O | N | $CH_3$ | $-O-CH_2-CF_3$ | 110–112° |
| 2 | H | H | O | $CHF_2$ | O | N | $C_2H_5$ | $-O-CH_2-CF_3$ | 129–131° |
| 3 | H | H | O | $CHF_2$ | O | N | $OC_2H_5$ | $-O-CH_2-CF_3$ | 147–148° |
| 4 | H | H | O | $CHF_2$ | S | N | $OCH_3$ | $-O-CH_2-CF_3$ | |
| 5 | H | H | S | $CHF_2$ | O | N | $OCH_3$ | $-O-CH_2-CF_3$ | 152–154° |
| 6 | H | H | O | $CHF_2$ | O | N | $OCH_3$ | $-O-CH_2-CF_3$ | 155–157° |
| 7 | H | H | O | $-CF_2-CHF_2$ | O | N | $OCH_3$ | $-O-CH_2-CF_3$ | 169–170° |
| 8 | H | H | O | $CF_3$ | O | N | $OCH_3$ | $-O-CH_2-CF_3$ | 157–159° |
| 9 | H | H | O | $CHF_2$ | O | CH | $OCH_3$ | $-O-CH_2-CF_3$ | 164–165° |
| 10 | H | H | O | $CHF_2$ | O | CH | $CH_3$ | $-O-CH_2-CF_3$ | 157–158° |
| 11 | H | H | O | $CHF_2$ | O | CH | Cl | $-O-CH_2-CF_3$ | 147–148° |
| 12 | H | H | O | $CHF_2$ | O | CH | $SCH_3$ | $-O-CH_2-CF_3$ | |
| 13 | H | H | O | $CHF_2$ | O | CH | $CH_2Cl$ | $-O-CH_2-CF_3$ | 114–118° |
| 14 | H | H | S | $CHF_2$ | O | CH | $OCH_3$ | $-O-CH_2-CF_3$ | 169–170° |

Fortsetzung

| Nr. | $R_1$ | $R_2$ | X | A | Z | E | $R_3$ | $R_4$ | Smp. [°C] |
|---|---|---|---|---|---|---|---|---|---|
| 15 | H | H | O | $CHF_2$ | O | N | $OCH_3$ | $-O-CH_2-CH_2Cl$ | 119–120° |
| 16 | H | H | O | $CHF_2$ | O | N | $OCH_3$ | $-O-CH_2-CH_2F$ | 94– 95° |
| 17 | H | H | O | $CHF_2$ | O | N | $OCH_3$ | $-O-CH_2-CH_2Br$ | |
| 18 | H | H | O | $CHF_2$ | O | CH | $OCH_3$ | $-O-CH_2-CH_2Cl$ | |
| 19 | H | H | O | $CHF_2$ | O | CH | $OCH_3$ | $-O-CH_2-CH_2F$ | 88° (Zers.) |
| 20 | 5–F | H | O | $CHF_2$ | O | N | $OCH_3$ | $-O-CH_2-CF_3$ | 117–119° |
| 21 | H | 6–Cl | O | $CHF_2$ | O | N | $OCH_3$ | $-O-CH_2-CF_3$ | 126–127° |
| 22 | 5–F | H | O | $CHF_2$ | O | CH | $OCH_3$ | $-O-CH_2-CF_3$ | |
| 23 | 5–F | H | O | $CHF_2$ | O | CH | $OCH_3$ | $-O-CH_2-CH_2Cl$ | 141–148° |
| 24 | H | H | O | $-CH_2-CH{=}CH_2$ | O | N | $OCH_3$ | $-O-CH_2-CF_3$ | |
| 25 | H | H | O | $-CH_2-CH_2Cl$ | O | N | $OCH_3$ | $-O-CH_2-CF_3$ | 135–137° |
| 26 | H | H | O | $-CCl{=}CHCl$ | O | N | $OCH_3$ | $-O-CH_2-CF_3$ | 133–134° |
| 27 | H | H | O | $-CH_2-CH_2-OCH_3$ | O | N | $OCH_3$ | $-O-CH_2-CF_3$ | 146–148° |
| 28 | H | H | O | $-CH_2-OCH_3$ | O | N | $OCH_3$ | $-O-CH_2-CF_3$ | |
| 29 | 5–Cl | H | O | $-CH_2-CH{=}CH_2$ | O | N | $OCH_3$ | $-O-CH_2-CF_3$ | |
| 30 | 5–F | H | O | $-CH_2-CH{=}CH_2$ | O | N | $OCH_3$ | $-O-CH_2-CF_3$ | |
| 31 | H | H | O | $CHF_2$ | O | CH | $CH_3$ | $-O-CHF_2$ | 161–162° |
| 32 | H | H | O | $CHF_2$ | O | CH | $CF_3$ | $OCHF_2$ | 130–131° |
| 33 | H | H | O | $CHF_2$ | O | CH | $OCH_3$ | $OCHF_3$ | 168–169° |

Fortsetzung

| Nr. | $R_1$ | $R_2$ | X | A | Z | E | $R_3$ | $R_4$ | Smp. [°C] |
|---|---|---|---|---|---|---|---|---|---|
| 34 | H | H | O | $CHF_2$ | O | CH | Cl | $OCHF_2$ | 133–134° |
| 35 | H | H | O | $CHF_2$ | O | CH | $C_2H_5$ | $OCHF_2$ | 145–146° |
| 36 | H | H | O | $CHF_2$ | O | CH | $SCH_3$ | $OCHF_2$ | |
| 37 | H | H | O | $CHF_2$ | O | CH | $CH_2F$ | $OCHF_2$ | |
| 40 | H | H | O | $CHF_2$ | O | N | $OCH_3$ | $OCHF_2$ | |
| 41 | H | H | O | $CHF_2$ | O | N | $-O-CH_2-CF_3$ | $-O-CH_2-CF_3$ | |
| 42 | H | H | O | $CHF_2$ | O | N | $OCH_3$ | $-O-CH_2-CHCl_2$ | |
| 43 | H | H | O | $CHF_2$ | O | N | $OCH_3$ | $-O-CH_2-CHCl-CH_2Cl$ | |
| 44 | H | H | O | $CHF_2$ | O | N | Cl | $-O-CH_2-CF_3$ | |
| 45 | H | H | O | $CHF_2$ | O | CH | $-O-CH_2-CF_3$ | $OCHF_2$ | |
| 46 | H | H | O | $-CH_2-OCH_3$ | O | CH | $CH_3$ | $OCHF_2$ | |
| 47 | H | H | O | $-CH_2-OCH_3$ | O | CH | $CF_3$ | $OCHF_2$ | |
| 48 | H | H | O | $-CH_2-OCH_3$ | O | CH | Cl | $OCHF_2$ | |
| 49 | H | H | O | $-CH_2-OCH_3$ | O | CH | $OCH_3$ | $OCHF_2$ | |
| 50 | H | H | O | $-CH_2-OCH_3$ | O | CH | $C_2H_5$ | $OCHF_2$ | |
| 51 | H | H | O | $-CH_2-OCH_3$ | O | CH | $CH_2F$ | $OCHF_2$ | |
| 52 | H | H | O | $-CH_2-OCH_3$ | O | CH | $SCH_3$ | $OCHF_2$ | |
| 54 | H | H | O | $-CH_2-OCH_3$ | O | N | $CH_3$ | $OCHF_2$ | |
| 55 | H | H | O | $-CH_2-OCH_3$ | O | N | $OCH_3$ | $-O-CH_2-CH_2Cl$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 56 | H | H | O | $-CH_2-OCH_3$ | O | N | $OCH_3$ | $-O-CH_2-CH_2F$ | |
| 57 | H | H | O | $-CH_2-OCH_3$ | O | N | $OCH_3$ | $-O-CH_2-CHCl_2$ | |
| 58 | H | H | O | $-CH_2-OCH_3$ | O | N | $OCH_3$ | $-O-CH_2-CHCl-CH_2Cl$ | |
| 59 | H | H | O | $-CH_2-OCH_3$ | O | N | $CH_3$ | $-O-CH_2-CF_3$ | |
| 60 | H | H | O | $-CH_2-OCH_3$ | O | CH | $CH_3$ | $-O-CH_2-CF_3$ | |
| 61 | H | H | O | $-CH_2-OCH_3$ | O | CH | Cl | $-O-CH_2-CF_3$ | |
| 62 | H | H | O | $-CH_2-OCH_3$ | O | CH | $OCH_3$ | $-O-CH_2-CF_3$ | |
| 63 | H | H | O | $-CH_2-CH=CH_2$ | O | CH | $CH_3$ | $OCHF_2$ | 180–184° |
| 64 | H | H | O | $-CH_2-CH=CH_2$ | O | CH | $CF_3$ | $OCHF_2$ | |
| 65 | H | H | O | $-CH_2-CH=CH_2$ | O | CH | Cl | $OCHF_2$ | |
| 66 | H | H | O | $-CH_2-CH=CH_2$ | O | CH | $OCH_3$ | $OCHF_2$ | |
| 67 | H | H | O | $-CH_2-CH=CH_2$ | O | CH | $C_2H_5$ | $OCHF_2$ | |
| 68 | H | H | O | $-CH_2-CH=CH_2$ | O | CH | $CH_2F$ | $OCHF_2$ | |
| 69 | H | H | O | $-CH_2-CH=CH_2$ | O | CH | $SCH_3$ | $OCHF_2$ | |
| 71 | H | H | O | $-CH_2-CH=CH_2$ | O | N | $CH_3$ | $OCHF_2$ | |
| 72 | H | H | O | $-CH_2-CH=CH_2$ | O | N | $OCH_3$ | $-O-CH_2-CH_2Cl$ | |
| 73 | H | H | O | $-CH_2-CH=CH_2$ | O | N | $OCH_3$ | $-O-CH_2-CH_2F$ | |
| 74 | H | H | O | $-CH_2-CH=CH_2$ | O | N | $OCH_3$ | $-O-CH_2-CHCl_2$ | |
| 75 | H | H | O | $-CH_2-CH=CH_2$ | O | N | $OCH_3$ | $-O-CH_2-CHCl-CH_2Cl$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 76 | H | H | O | $-CH_2-CH=CH_2$ | O | N | $CH_3$ | $-O-CH_2-CF_3$ | |
| 77 | H | H | O | $-CH_2-CH=CH_2$ | O | CH | $CH_3$ | $-O-CH_2-CF_3$ | |
| 78 | H | H | O | $-CH_2-CH=CH_2$ | O | CH | Cl | $-O-CH_2-CF_3$ | |
| 79 | H | H | O | $-CH_2-CH=CH_2$ | O | CH | $OCH_3$ | $-O-CH_2-CF_3$ | 190–200° |
| 80 | H | H | O | $-CF_2-CF_3$ | O | CH | $CH_3$ | $OCHF_2$ | 185–186° |
| 81 | H | H | O | $-CF_2-CF_3$ | O | CH | $CF_3$ | $OCHF_2$ | |
| 82 | H | H | O | $-CF_2-CF_3$ | O | CH | Cl | $OCHF_2$ | |
| 83 | H | H | O | $-CF_2-CF_3$ | O | CH | $OCH_3$ | $OCHF_2$ | |
| 84 | H | H | O | $-CF_2-CF_3$ | O | CH | $C_2H_5$ | $OCHF_2$ | |
| 85 | H | H | O | $-CF_2-CF_3$ | O | CH | $CH_2F$ | $OCHF_2$ | |
| 86 | H | H | O | $-CF_2-CF_3$ | O | CH | $SCH_3$ | $OCHF_2$ | |
| 88 | H | H | O | $-CF_2-CF_3$ | O | N | $CH_3$ | $OCHF_2$ | |
| 89 | H | H | O | $-CF_2-CF_3$ | O | N | $OCH_3$ | $-O-CH_2-CH_2Cl$ | |
| 90 | H | H | O | $-CF_2-CF_3$ | O | N | $OCH_3$ | $-O-CH_2-CH_2F$ | |
| 91 | H | H | O | $-CF_2-CF_3$ | O | N | $OCH_3$ | $-O-CH_2-CHCl_2$ | |
| 92 | H | H | O | $-CF_2-CF_3$ | O | N | $OCH_3$ | $-O-CH_2-CHCl-CH_2Cl$ | |
| 93 | H | H | O | $-CF_2-CF_3$ | O | N | $CH_3$ | $-O-CH_2CF_3$ | |
| 94 | H | H | O | $-CF_2-CF_3$ | O | CH | $CH_3$ | $-O-CH_2CF_3$ | |
| 95 | H | H | O | $-CF_2-CF_3$ | O | CH | Cl | $-O-CH_2-CF_3$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 96 | H | H | O | $-CF_2-CF_3$ | O | CH | $OCH_3$ | $-O-CH_2-CF_3$ | 166–167° |
| 97 | H | H | O | $-CF_2-CF_3$ | O | N | $OCH_3$ | $-O-CH_2-CF_3$ | 182–183° |
| 98 | H | H | O | $-CH_2-CH_2-OCH_3$ | O | CH | $CH_3$ | $OCHF_2$ | 148–149° |
| 99 | H | H | O | $-CH_2-CH_2-OCH_3$ | O | CH | $CF_3$ | $OCHF_2$ | |
| 100 | H | H | O | $-CH_2-CH_2-OCH_3$ | O | CH | Cl | $OCHF_2$ | 142–143° |
| 101 | H | H | O | $-CH_2-CH_2-OCH_3$ | O | CH | $OCH_3$ | $OCHF_2$ | 157–158° |
| 102 | H | H | O | $-CH_2-CH_2-OCH_3$ | O | CH | $C_2F_5$ | $OCHF_2$ | |
| 103 | H | H | O | $-CH_2-CH_2-OCH_3$ | O | CH | $CH_2F$ | $OCHF_2$ | |
| 104 | H | H | O | $-CH_2-CH_2-OCH_3$ | O | CH | $SCH_3$ | $OCHF_2$ | |
| 106 | H | H | O | $-CH_2-CH_2-OCH_3$ | O | N | $CH_3$ | $OCHF_2$ | |
| 107 | H | H | O | $-CH_2-CH_2-OCH_3$ | O | N | $OCH_3$ | $-O-CH_2-CH_2Cl$ | |
| 108 | H | H | O | $-CH_2-CH_2-OCH_3$ | O | N | $OCH_3$ | $-O-CH_2-CH_2F$ | |
| 109 | H | H | O | $-CH_2-CH_2-OCH_3$ | O | N | $OCH_3$ | $-O-CH_2-CHCl_2$ | |
| 110 | H | H | O | $-CH_2-CH_2-OCH_3$ | O | N | $OCH_3$ | $-O-CH_2-CHCl-CH_2Cl$ | |
| 111 | H | H | O | $-CH_2-CH_2-OCH_3$ | O | N | $CH_3$ | $-O-CH_2-CF_3$ | |
| 112 | H | H | O | $-CH_2-CH_2-OCH_3$ | O | CH | $CH_3$ | $-O-CH_2-CF_3$ | |
| 113 | H | H | O | $-CH_2-CH_2-OCH_3$ | O | CH | Cl | $-O-CH_2-CF_3$ | |
| 114 | H | H | O | $-CH_2-CH_2-OCH_3$ | O | CH | $OCH_3$ | $-O-CH_2-CF_3$ | |
| 115 | H | H | O | $-CH_2-CH_2-Cl$ | O | CH | $CH_3$ | $OCHF_2$ | 182–183° |

Fortsetzung

| Nr. | $R_1$ | $R_2$ | X | A | Z | E | $R_3$ | $R_4$ | Smp. [° C] |
|---|---|---|---|---|---|---|---|---|---|
| 116 | H | H | O | $-CH_2-CH_2Cl$ | O | CH | $CF_3$ | $OCHF_2$ | |
| 117 | H | H | O | $-CH_2-CH_2Cl$ | O | CH | Cl | $OCHF_2$ | |
| 118 | H | H | O | $-CH_2-CH_2Cl$ | O | CH | $OCH_3$ | $OCHF_2$ | |
| 119 | H | H | O | $-CH_2-CH_2Cl$ | O | CH | $C_2H_5$ | $OCHF_2$ | |
| 120 | H | H | O | $-CH_2-CH_2Cl$ | O | CH | $CH_2F$ | $OCHF_2$ | |
| 121 | H | H | O | $-CH_2-CH_2Cl$ | O | CH | $SCH_3$ | $OCHF_2$ | |
| 123 | H | H | O | $-CH_2-CH_2Cl$ | O | N | $CH_3$ | $OCHF_2$ | |
| 124 | H | H | O | $-CH_2-CH_2Cl$ | O | N | $OCH_3$ | $-O-CH_2-CH_2Cl$ | |
| 125 | H | H | O | $-CH_2-CH_2Cl$ | O | N | $OCH_3$ | $-O-CH_2-CH_2F$ | |
| 126 | H | H | O | $-CH_2-CH_2Cl$ | O | N | $OCH_3$ | $-O-CH_2-CHCl_2$ | |
| 127 | H | H | O | $-CH_2-CH_2Cl$ | O | N | $OCH_3$ | $-O-CH_2-CHCl-CH_2Cl$ | |
| 128 | H | H | O | $-CH_2-CH_2Cl$ | O | N | $CH_3$ | $-O-CH_2-CF_3$ | |
| 129 | H | H | O | $-CH_2-CH_2Cl$ | O | CH | $CH_3$ | $-O-CH_2-CF_3$ | |
| 130 | H | H | O | $-CH_2-CH_2Cl$ | O | CH | Cl | $-O-CH_2-CF_3$ | |
| 131 | H | H | O | $-CH_2-CH_2Cl$ | O | CH | $OCH_3$ | $-O-CH_2-CF_3$ | |
| 132 | H | H | O | $-CCl=CHCl$ | O | CH | $CH_3$ | $OCHF_2$ | 216–217° (Zers.) |
| 133 | H | H | O | $-CCl=CHCl$ | O | CH | $CF_3$ | $OCHF_2$ | |
| 134 | H | H | O | $-CCl=CHCl$ | O | CH | Cl | $OCHF_2$ | 195–196° |
| 135 | H | H | O | $-CCl=CHCl$ | O | CH | $OCH_3$ | $OCHF_2$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 136 | H | H | O | $-CCl{=}CHCl$ | O | CH | $C_2H_5$ | $OCHF_2$ | |
| 137 | H | H | O | $-CCl{=}CHCl$ | O | CH | $CH_2F$ | $OCHF_2$ | |
| 138 | H | H | O | $-CCl{=}CHCl$ | O | CH | $SCH_3$ | $OCHF_2$ | |
| 140 | H | H | O | $-CCl{=}CHCl$ | O | N | $CH_3$ | $OCHF_2$ | |
| 141 | H | H | O | $-CCl{=}CHCl$ | O | N | $OCH_3$ | $-O-CH_2-CH_2Cl$ | |
| 142 | H | H | O | $-CCl{=}CHCl$ | O | N | $OCH_3$ | $-O-CH_2-CH_2F$ | |
| 143 | H | H | O | $-CCl{=}CHCl$ | O | N | $OCH_3$ | $-O-CH_2-CHCl_2$ | |
| 144 | H | H | O | $-CCl{=}CHCl$ | O | N | $OCH_3$ | $-O-CH_2-CHCl-CH_2Cl$ | |
| 145 | H | H | O | $-CCl{=}CHCl$ | O | N | $CH_3$ | $-O-CH_2-CF_3$ | |
| 146 | H | H | O | $-CCl{=}CHCl$ | O | CH | $CH_3$ | $-O-CH_2-CF_3$ | |
| 147 | H | H | O | $-CCl{=}CHCl$ | O | CH | Cl | $-O-CH_2-CF_3$ | |
| 148 | H | H | O | $-CCl{=}CHCl$ | O | CH | $OCH_3$ | $-O-CH_2-CF_3$ | 193–194° |
| 149 | H | H | O | $-CH_2-CF_3$ | O | CH | $CH_3$ | $OCHF_2$ | |
| 150 | H | H | O | $-CH_2-CF_3$ | O | CH | $CF_3$ | $OCHF_2$ | |
| 151 | H | H | O | $-CH_2-CF_3$ | O | CH | Cl | $OCHF_2$ | |
| 152 | H | H | O | $-CF_2-CF_3$ | O | CH | $OCH_3$ | $OCHF_2$ | |
| 153 | H | H | O | $-CF_2-CF_3$ | O | CH | $C_2H_5$ | $OCHF_2$ | |
| 154 | H | H | O | $-CF_2-CF_3$ | O | CH | $CH_2F$ | $OCHF_2$ | |
| 155 | H | H | O | $-CF_2-CF_3$ | O | CH | $SCH_3$ | $OCHF_2$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 157 | H | H | O | $-CH_2-CF_3$ | O | N | $CH_3$ | $OCHF_2$ | |
| 158 | H | H | O | $-CH_2-CF_3$ | O | N | $OCH_3$ | $-O-CH_2-CH_2Cl$ | |
| 159 | H | H | O | $-CH_2-CF_3$ | O | N | $OCH_3$ | $-O-CH_2-CH_2F$ | |
| 160 | H | H | O | $-CH_2-CF_3$ | O | N | $OCH_3$ | $-O-CH_2-CHCl_2$ | |
| 161 | H | H | O | $-CH_2-CF_3$ | O | N | $OCH_3$ | $-O-CH_2-CHCl-CH_2Cl$ | |
| 162 | H | H | O | $-CH_2-CF_3$ | O | N | $CH_3$ | $-O-CH_2-CF_3$ | |
| 163 | H | H | O | $-CH_2-CF_3$ | O | CH | $CH_3$ | $-O-CH_2-CF_3$ | |
| 164 | H | H | O | $-CH_2-CF_3$ | O | CH | Cl | $-O-CH_2-CF_3$ | |
| 165 | H | H | O | $-CH_2-CF_3$ | O | CH | $OCH_3$ | $-O-CH_2-CF_3$ | |
| 166 | H | H | O | $-CH_2-CF_3$ | O | N | $OCH_3$ | $-O-CH_2-CF_3$ | |
| 167 | H | H | O | $-CF_2-CHF_2$ | O | CH | $CH_3$ | $OCHF_2$ | 174–175° |
| 168 | H | H | O | $-CF_2-CHF_2$ | O | CH | $CF_3$ | $OCHF_2$ | |
| 169 | H | H | O | $-CF_2-CHF_2$ | O | CH | Cl | $OCHF_2$ | |
| 170 | H | H | O | $-CF_2-CHF_2$ | O | CH | $OCH_3$ | $OCHF_2$ | |
| 171 | H | H | O | $-CF_2-CHF_2$ | O | CH | $C_2H_5$ | $OCHF_2$ | |
| 172 | H | H | O | $-CF_2-CHF_2$ | O | CH | $CH_2F$ | $OCHF_2$ | |
| 173 | H | H | O | $-CF_2-CHF_2$ | O | CH | $SCH_3$ | $OCHF_2$ | |
| 175 | H | H | O | $-CF_2-CHF_2$ | O | N | $CH_3$ | $OCHF_2$ | |
| 176 | H | H | O | $-CF_2-CHF_2$ | O | N | $OCH_3$ | $-O-CH_2-CH_2Cl$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 177 | H | H | O | $-CF_2-CHF_2$ | O | N | $OCH_3$ | $-O-CH_2-CH_2F$ | |
| 178 | H | H | O | $-CF_2-CHF_2$ | O | N | $OCH_3$ | $-O-CH_2-CHCl_2$ | |
| 179 | H | H | O | $-CF_2-CHF_2$ | O | N | $OCH_3$ | $-O-CH_2-CHCl-CH_2Cl$ | |
| 180 | H | H | O | $-CF_2-CHF_2$ | O | N | $CH_3$ | $-O-CH_2-CF_3$ | |
| 181 | H | H | O | $-CF_2-CHF_2$ | O | CH | $CH_3$ | $-O-CH_2-CF_3$ | |
| 182 | H | H | O | $-CF_2-CHF_2$ | O | CH | Cl | $-O-CH_2-CF_3$ | |
| 183 | H | H | O | $-CF_2-CHF_2$ | O | CH | $OCH_3$ | $-O-CH_2-CF_2$ | |
| 184 | H | H | O | $CF_3$ | O | CH | $CH_3$ | $OCHF_2$ | 178–179° |
| 185 | H | H | O | $CF_3$ | O | CH | $CF_3$ | $OCHF_2$ | |
| 186 | H | H | O | $CF_3$ | O | CH | Cl | $OCHF_2$ | 139–141° |
| 187 | H | H | O | $CF_3$ | O | CH | $OCH_3$ | $OCHF_2$ | 186–187° |
| 188 | H | H | O | $CF_3$ | O | CH | $C_2H_5$ | $OCHF_2$ | |
| 189 | H | H | O | $CF_3$ | O | CH | $CH_2F$ | $OCHF_2$ | |
| 190 | H | H | O | $CF_3$ | O | CH | $SCH_3$ | $OCHF_2$ | |
| 192 | H | H | O | $CF_3$ | O | N | $CH_3$ | $OCHF_2$ | |
| 193 | H | H | O | $CF_3$ | O | N | $OCH_3$ | $-O-CH_2-CH_2Cl$ | |
| 194 | H | H | O | $CF_3$ | O | N | $OCH_3$ | $-O-CH_2-CH_2F$ | |
| 195 | H | H | O | $CF_3$ | O | N | $OCH_3$ | $-O-CH_2-CHCl_2$ | |
| 196 | H | H | O | $CF_3$ | O | N | $OCH_3$ | $-O-CH_2-CHCl-CH_2Cl$ | |

Fortsetzung

| Nr. | $R_1$ | $R_2$ | X | A | Z | E | $R_3$ | $R_4$ | S[redacted]mp. [°C] |
|---|---|---|---|---|---|---|---|---|---|
| 197 | H | H | O | $CF_3$ | O | N | $CH_3$ | $-O-CH_2-CF_3$ | |
| 198 | H | H | O | $CF_3$ | O | CH | $CH_3$ | $-O-CH_2-CF_3$ | |
| 199 | H | H | O | $CF_3$ | O | CH | Cl | $-O-CH_2-CF_3$ | |
| 200 | H | H | O | $CF_3$ | O | CH | $OCH_3$ | $-O-CH_2-CF_3$ | |
| 201 | H | H | S | $CHF_2$ | O | CH | $CH_3$ | $OCHF_2$ | 1[redacted]59–160° |
| 202 | H | H | S | $CHF_2$ | O | CH | $CF_3$ | $OCHF_2$ | |
| 203 | H | H | S | $CHF_2$ | O | CH | Cl | $OCHF_2$ | |
| 204 | H | H | S | $CHF_2$ | O | CH | $OCH_3$ | $OCHF_2$ | 1—44–146° |
| 205 | H | H | S | $CHF_2$ | O | CH | $C_2H_5$ | $OCHF_2$ | |
| 206 | H | H | S | $CHF_2$ | O | CH | $CH_2F$ | $OCHF_2$ | |
| 207 | H | H | S | $CHF_2$ | O | CH | $SCH_3$ | $OCHF_2$ | |
| 209 | H | H | S | $CHF_2$ | O | N | $CH_3$ | $OCHF_2$ | |
| 210 | H | H | S | $CHF_2$ | O | N | $OCH_3$ | $-O-CH_2-CH_2Cl$ | |
| 211 | H | H | S | $CHF_2$ | O | N | $OCH_3$ | $-O-CH_2-CH_2F$ | |
| 212 | H | H | S | $CHF_2$ | O | N | $OCH_3$ | $-O-CH_2-CHCl_2$ | |
| 213 | H | H | S | $CHF_2$ | O | N | $OCH_3$ | $-O-CH_2-CHCl-CH_2Cl$ | |
| 214 | H | H | S | $CHF_2$ | O | N | $CH_3$ | $-O-CH_2-CF_3$ | |
| 215 | H | H | S | $CHF_2$ | O | CH | $CH_3$ | $-O-CH_2-CF_3$ | |
| 216 | H | H | S | $CHF_2$ | O | CH | Cl | $-O-CH_2-CF_3$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 217 | H | H | $SO_2$ | $CHF_2$ | O | CH | $CH_3$ | $OCHF_2$ | 184–186° |
| 218 | H | H | $SO_2$ | $CHF_2$ | O | CH | $CF_3$ | $OCHF_2$ | |
| 219 | H | H | $SO_2$ | $CHF_2$ | O | CH | Cl | $OCHF_2$ | |
| 220 | H | H | $SO_2$ | $CHF_2$ | O | CH | $OCH_3$ | $OCHF_2$ | |
| 221 | H | H | $SO_2$ | $CHF_2$ | O | CH | $C_2H_5$ | $OCHF_2$ | |
| 222 | H | H | $SO_2$ | $CHF_2$ | O | CH | $CH_2F$ | $OCHF_2$ | |
| 223 | H | H | $SO_2$ | $CHF_2$ | O | CH | $SCH_3$ | $OCHF_2$ | |
| 225 | H | H | $SO_2$ | $CHF_2$ | O | N | $CH_3$ | $OCHF_2$ | |
| 226 | H | H | $SO_2$ | $CHF_2$ | O | N | $OCH_3$ | $-O-CH_2-CH_2Cl$ | |
| 227 | H | H | $SO_2$ | $CHF_2$ | O | N | $OCH_3$ | $-O-CH_2-CH_2F$ | |
| 228 | H | H | $SO_2$ | $CHF_2$ | O | N | $OCH_3$ | $-O-CH_2-CHCl_2$ | |
| 229 | H | H | $SO_2$ | $CHF_2$ | O | N | $OCH_3$ | $-O-CH_2-CHCl-CH_2Cl$ | |
| 230 | H | H | $SO_2$ | $CHF_2$ | O | N | $CH_3$ | $-O-CH_2-CF_3$ | |
| 231 | H | H | $SO_2$ | $CHF_2$ | O | CH | $CH_3$ | $-O-CH_2-CF_3$ | |
| 232 | H | H | $SO_2$ | $CHF_2$ | O | CH | Cl | $-O-CH_2-CF_3$ | |
| 233 | H | H | $SO_2$ | $CHF_2$ | O | CH | $OCH_3$ | $-O-CH_2-CF_3$ | |
| 234 | H | H | $SO_2$ | $CHF_2$ | O | N | $OCH_3$ | $-O-CH_2-CF_3$ | |
| 235 | 5-F | H | O | $-CH_2-CH=CH_2$ | O | CH | $CH_3$ | $OCHF_2$ | |
| 236 | 5-F | H | O | $-CH_2-CH=CH_2$ | O | CH | $CF_3$ | $OCHF_2$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 237 | 5-F | H | O | $-CH_2-CH=CH_2$ | O | CH | Cl | $OCHF_2$ | |
| 238 | 5-F | H | O | $-CH_2-CH=CH_2$ | O | CH | $OCH_3$ | $OCHF_2$ | |
| 239 | 5-F | H | O | $-CH_2-CH=CH_2$ | O | CH | $C_2H_5$ | $OCHF_2$ | |
| 240 | 5-F | H | O | $-CH_2-CH=CH_2$ | O | CH | $CH_2F$ | $OCHF_2$ | |
| 241 | 5-F | H | O | $-CH_2-CH=CH_2$ | O | CH | $SCH_3$ | $OCHF_2$ | |
| 243 | 5-F | H | O | $-CH_2-CH=CH_2$ | O | N | $CH_3$ | $OCHF_2$ | |
| 244 | 5-F | H | O | $-CH_2-CH=CH_2$ | O | N | $OCH_3$ | $-O-CH_2-CH_2Cl$ | |
| 245 | 5-F | H | O | $-CH_2-CH=CH_2$ | O | N | $OCH_3$ | $-O-CH_2-CH_2F$ | |
| 246 | 5-F | H | O | $-CH_2-CH=CH_2$ | O | N | $OCH_3$ | $-O-CH_2-CHCl_2$ | |
| 247 | 5-F | H | O | $-CH_2-CH=CH_2$ | O | N | $OCH_3$ | $-O-CH_2-CHCl-CH_2Cl$ | |
| 248 | 5-F | H | O | $-CH_2-CH=CH_2$ | O | N | $CH_3$ | $-O-CH_2-CF_3$ | |
| 249 | 5-F | H | O | $-CH_2-CH=CH_2$ | O | CH | $CH_3$ | $-O-CH_2-CF_3$ | |
| 250 | 5-F | H | O | $-CH_2-CH=CH_2$ | O | CH | Cl | $-O-CH_2-CF_3$ | |
| 251 | 5-F | H | O | $-CH_2-CH=CH_2$ | O | CH | $OCH_3$ | $-O-CH_2-CF_3$ | |
| 252 | 5-F | H | O | $CHF_2$ | O | CH | $CH_3$ | $OCHF_2$ | 150–151 |
| 253 | 5-F | H | O | $CHF_2$ | O | CH | $CF_3$ | $OCHF_2$ | |
| 254 | 5-F | H | O | $CHF_2$ | O | CH | Cl | $OCHF_2$ | |
| 255 | 5-F | H | O | $CHF_2$ | O | CH | $OCH_3$ | $OCHF_2$ | |
| 256 | 5-F | H | O | $CHF_2$ | O | CH | $C_2H_5$ | $OCHF_2$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 257 | 5-F | H | O | $CHF_2$ | O | CH | $CH_2F$ | $OCHF_2$ | |
| 258 | 5-F | H | O | $CHF_2$ | O | CH | $SCH_3$ | $OCHF_2$ | |
| 260 | 5-F | H | O | $CHF_2$ | O | N | $CH_3$ | $OCHF_2$ | |
| 261 | 5-F | H | O | $CHF_2$ | O | N | $OCH_3$ | $-O-CH_2-CH_2Cl$ | |
| 262 | 5-F | H | O | $CHF_2$ | O | N | $OCH_3$ | $-O-CH_2-CH_2F$ | |
| 263 | 5-F | H | O | $CHF_2$ | O | N | $OCH_3$ | $-O-CH_2-CHCl_2$ | |
| 264 | 5-F | H | O | $CHF_2$ | O | N | $OCH_3$ | $-O-CH_2-CHCl-CH_2Cl$ | |
| 265 | 5-F | H | O | $CHF_2$ | O | N | $CH_3$ | $-O-CH_2-CF_3$ | |
| 266 | 5-F | H | O | $CHF_2$ | O | CH | $CH_3$ | $-O-CH_2-CF_3$ | |
| 267 | 5-F | H | O | $CHF_2$ | O | CH | Cl | $-O-CH_2-CF_3$ | |
| 268 | 5-F | H | O | $CHF_2$ | O | CH | $OCH_3$ | $-O-CH_2-CF_3$ | |
| 269 | H | 6-Cl | O | $CHF_2$ | O | CH | $CH_3$ | $OCHF_2$ | 103–104° |
| 270 | H | 6-Cl | O | $CHF_2$ | O | CH | $CF_3$ | $OCHF_2$ | |
| 271 | H | 6-Cl | O | $CHF_2$ | O | CH | Cl | $OCHF_2$ | |
| 272 | H | 6-Cl | O | $CHF_2$ | O | CH | $OCH_3$ | $OCHF_2$ | 112–114° |
| 273 | H | 6-Cl | O | $CHF_2$ | O | CH | $C_2H_5$ | $OCHF_2$ | |
| 274 | H | 6-Cl | O | $CHF_2$ | O | CH | $CH_2F$ | $OCHF_2$ | |
| 275 | H | 6-Cl | O | $CHF_2$ | O | CH | $SCH_3$ | $OCHF_2$ | |
| 277 | H | 6-Cl | O | $CHF_2$ | O | N | $CH_3$ | $OCHF_2$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 278 | H | 6-Cl | O | $CHF_2$ | O | N | $OCH_3$ | $-O-CH_2-CH_2Cl$ | |
| 279 | H | 6-Cl | O | $CHF_2$ | O | N | $OCH_3$ | $-O-CH_2-CH_2F$ | |
| 280 | H | 6-Cl | O | $CHF_2$ | O | N | $OCH_3$ | $-O-CH_2-CHCl_2$ | |
| 281 | H | 6-Cl | O | $CHF_2$ | O | N | $OCH_3$ | $-O-CH_2-CHCl-CH_2Cl$ | |
| 282 | H | 6-Cl | O | $CHF_2$ | O | N | $CH_3$ | $-O-CH_2-CF_3$ | |
| 283 | H | 6-Cl | O | $CHF_2$ | O | CH | $CH_3$ | $-O-CH_2-CF_3$ | |
| 284 | H | 6-Cl | O | $CHF_2$ | O | CH | Cl | $-O-CH_2-CF_3$ | |
| 285 | H | 6-Cl | O | $CHF_2$ | O | CH | $OCH_3$ | $-O-CH_2-CF_3$ | |
| 286 | H | H | O | $-CH_2-CH_2-O-C_2H_5$ | O | N | $OCH_3$ | $-O-CH_2-CF_3$ | 136–138° |
| 287 | H | H | O | $-CH_2-CH_2-O-C_2H_5$ | O | CH | $OCH_3$ | $OCHF_2$ | |
| 288 | H | H | O | $-CH_2-CH_2-O-C_2H_5$ | O | CH | $CH_3$ | $OCHF_2$ | 138–140° |
| 289 | H | H | O | $-CH_2-CH_2-O-C_2H_5$ | O | CH | Cl | $OCHF_2$ | |
| 290 | H | H | O | $-CH_2-CH_2-O-C_2H_5$ | O | CH | $CH_3$ | $-O-CH_2-CF_3$ | |
| 291 | H | H | O | $-CF_2-CHFCl$ | O | N | $OCH_3$ | $-O-CH_2-CF_3$ | 173–174° |
| 292 | H | H | O | $-CF_2-CHFCl$ | O | CH | $OCH_3$ | $OCHF_2$ | |
| 293 | H | H | O | $-CH_2-CHFCl$ | O | CH | $CH_3$ | $OCHF_2$ | 157–158° |
| 294 | H | H | O | $-CF_2-CHFCl$ | O | CH | Cl | $OCHF_2$ | |
| 295 | H | H | O | $-CF_2-CHFCl$ | O | CH | $CH_3$ | $-O-CH_2-CF_3$ | |
| 296 | H | H | O | $-CH_2-C(CH_3)=CH_2$ | O | N | $OCH_3$ | $-O-CH_2-CF_3$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 297 | H | H | O | $-CH_2-C(CH_3)=CH_2$ | O | CH | $OCH_3$ | $OCHF_2$ | |
| 298 | H | H | O | $-CH_2-C(CH_3)=CH_2$ | O | CH | $CH_3$ | $OCHF_2$ | 149–151° |
| 299 | H | H | O | $-CH_2-C(CH_3)=CH_2$ | O | CH | Cl | $OCHF_2$ | |
| 300 | H | H | O | $-CH_2-C(CH_3)=CH_2$ | O | CH | $CH_3$ | $-O-CH_2-CF_3$ | |
| 301 | H | H | O | $-CH_2-C(CH_3)=CH_2$ | O | CH | $OCH_3$ | $-O-CH_2-CF_3$ | 218–219° |
| 302 | H | H | O | $-CH_2-CHCl-CH_2Cl$ | O | N | $OCH_3$ | $-O-CH_2-CF_3$ | |
| 303 | H | H | O | $-CH_2-CHCl-CH_2Cl$ | O | CH | $OCH_3$ | $OCHF_2$ | |
| 304 | H | H | O | $-CH_2-CHCl-CH_2Cl$ | O | CH | $CH_3$ | $OCHF_2$ | |
| 305 | H | H | O | $-CH_2-CHCl-CH_2Cl$ | O | CH | Cl | $OCHF_2$ | |
| 306 | H | H | O | $-CH_2-CHCl-CH_2Cl$ | O | CH | $CH_3$ | $-O-CH_2-CF_3$ | |
| 307 | H | H | O | $-CH(CH_2Cl)_2$ | O | N | $OCH_3$ | $-O-CH_2-CF_3$ | |
| 308 | H | H | O | $-CH(CH_2Cl)_2$ | O | CH | $OCH_3$ | $OCHF_2$ | |
| 309 | H | H | O | $-CH(CH_2Cl)_2$ | O | CH | $CH_3$ | $OCHF_2$ | |
| 310 | H | H | O | $-CH(CH_2Cl)_2$ | O | CH | Cl | $OCHF_2$ | |
| 311 | H | H | O | $-CH(CH_2Cl)_2$ | O | CH | $CH_3$ | $-O-CH_2-CF_3$ | |
| 312 | H | H | O | $-CH_2-CH=C(CH_3)_2$ | O | N | $OCH_3$ | $-O-CH_2-CF_3$ | |
| 313 | H | H | O | $-CH_2-CH=C(CH_3)_2$ | O | CH | $OCH_3$ | $OCHF_2$ | |
| 314 | H | H | O | $-CH_2-CH=C(CH_3)_2$ | O | CH | $CH_3$ | $OCHF_2$ | |
| 315 | H | H | O | $-CH_2-CH=C(CH_3)_2$ | O | CH | Cl | $OCHF_2$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 316 | H | H | O | $-CH_2-CH=C(CH_3)_2$ | O | CH | $CH_3$ | $-O-CH_2-CF_3$ | |
| 317 | H | H | O | $-CH_2-CH_2F$ | O | N | $OCH_3$ | $-O-CH_2-CF_3$ | |
| 318 | H | H | O | $-CH_2-CH_2F$ | O | CH | $OCH_3$ | $OCHF_2$ | |
| 319 | H | H | O | $-CH_2-CH_2F$ | O | CH | $CH_3$ | $OCHF_2$ | |
| 320 | H | H | O | $-CH_2-CH_2F$ | O | CH | Cl | $OCHF_2$ | |
| 321 | H | H | O | $-CH_2-CH_2F$ | O | CH | $CH_3$ | $-O-CH_2-CF_3$ | |
| 322 | H | H | O | $-CH_2-CHCl_2$ | O | N | $OCH_3$ | $-O-CH_2-CF_3$ | |
| 323 | H | H | O | $-CH_2-CHCl_2$ | O | CH | $OCH_3$ | $OCHF_2$ | |
| 324 | H | H | O | $-CH_2-CHCl_2$ | O | CH | $CH_3$ | $OCHF_2$ | |
| 325 | H | H | O | $-CH_2-CHCl_2$ | O | CH | Cl | $OCHF_2$ | |
| 326 | H | H | O | $-CH_2-CHCl_2$ | O | CH | $CH_3$ | $-O-CH_2-CF_3$ | |
| 327 | H | H | O | $-CH_2-CCl_3$ | O | N | $OCH_3$ | $-O-CH_2-CF_3$ | |
| 328 | H | H | O | $-CH_2-CCl_3$ | O | CH | $OCH_3$ | $OCHF_2$ | |
| 329 | H | H | O | $-CH_2-CCl_3$ | O | CH | $CH_3$ | $OCHF_2$ | |
| 330 | H | H | O | $-CH_2-CCl_3$ | O | CH | Cl | $OCHF_2$ | |
| 331 | H | H | O | $-CH_2-CCl_3$ | O | CH | $CH_3$ | $-O-CH_2-CF_3$ | |
| 332 | H | H | S | $-CF_2-CHF_2$ | O | N | $OCH_3$ | $-O-CH_2-CF_3$ | |
| 333 | H | H | S | $-CF_2-CHF_2$ | O | CH | $OCH_3$ | $OCHF_2$ | |
| 334 | H | H | S | $-CF_2-CHF_2$ | O | CH | $CH_3$ | $OCHF_2$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 335 | H | H | S | $-CF_2-CHF_2$ | O | CH | Cl | $OCHF_2$ | |
| 336 | H | H | S | $-CF_2-CHF_2$ | O | CH | $CH_3$ | $-O-CH_2-CF_3$ | |
| 337 | H | H | O | $-CH_2-CH{=}CH-CH_3$ | O | N | $OCH_3$ | $-O-CH_2-CF_3$ | 148–153° |
| 338 | H | H | O | $-CH_2-CH{=}CH-CH_3$ | O | CH | $OCH_3$ | $OCHF_2$ | |
| 339 | H | H | O | $-CH_2-CH{=}CH-CH_3$ | O | CH | $CH_3$ | $OCHF_2$ | 171–172° |
| 340 | H | H | O | $-CH_2-CH{=}CH-CH_3$ | O | CH | Cl | $OCHF_2$ | |
| 341 | H | H | O | $-CH_2-CH{=}CH-CH_3$ | O | CH | $CH_3$ | $-O-CH_2-CF_3$ | |
| 342 | H | H | O | $-CH_2-CHCl-CHCl-CH_3$ | O | N | $OCH_3$ | $-O-CH_2-CF_3$ | |
| 343 | H | H | O | $-CH_2-CHCl-CHCl-CH_3$ | O | CH | $OCH_3$ | $OCHF_2$ | |
| 344 | H | H | O | $-CH_2-CHCl-CHCl-CH_3$ | O | CH | $CH_3$ | $OCHF_2$ | |
| 345 | H | H | O | $-CH_2-CHCl-CHCl-CH_3$ | O | CH | Cl | $OCHF_2$ | |
| 346 | H | H | O | $-CH_2-CHCl-CHCl-CH_3$ | O | CH | $CH_3$ | $-O-CH_2-CF_3$ | |
| 347 | H | H | O | $CF_2Cl$ | O | N | $OCH_3$ | $-O-CH_2-CF_3$ | |
| 348 | H | H | O | $CF_2Cl$ | O | CH | $OCH_3$ | $OCHF_2$ | |
| 349 | H | H | O | $CF_2Cl$ | O | CH | $CH_3$ | $OCHF_2$ | |
| 350 | H | H | O | $CF_2Cl$ | O | CH | Cl | $OCHF_2$ | |
| 351 | H | H | O | $CF_2Cl$ | O | CH | $CH_3$ | $-O-CH_2-CF_3$ | |
| 352 | H | H | O | $CF_2Br$ | O | N | $OCH_3$ | $-O-CH_2-CF_3$ | |
| 353 | H | H | O | $CF_2Br$ | O | CH | $OCH_3$ | $OCHF_2$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 354 | H | H | O | $CF_2Br$ | O | CH | $CH_3$ | $OCHF_2$ | |
| 355 | H | H | O | $CF_2Br$ | O | CH | Cl | $OCHF_2$ | |
| 356 | H | H | O | $CF_2Br$ | O | CH | $CH_3$ | $-O-CH_2-CF_3$ | |
| 357 | 5–Cl | H | O | $OCHF_2$ | O | N | $OCH_3$ | $-O-CH_2-CF_3$ | 92– 94 |
| 358 | 5–Cl | H | O | $OCHF_2$ | O | CH | $OCH_3$ | $OCHF_2$ | |
| 359 | 5–Cl | H | O | $OCHF_2$ | O | CH | $CH_3$ | $OCHF_2$ | 133–134 |
| 360 | 5–Cl | H | O | $OCHF_2$ | O | CH | Cl | $OCHF_2$ | |
| 361 | 5–Cl | H | O | $OCHF_2$ | O | CH | $CH_3$ | $-O-CH_2-CF_3$ | 153–155 |
| 362 | H | 6–F | O | $CHF_2$ | O | N | $OCH_3$ | $-O-CH_2-CF_3$ | |
| 363 | H | 6–F | O | $CHF_2$ | O | CH | $OCH_3$ | $OCHF_2$ | |
| 364 | H | 6–F | O | $CHF_2$ | O | CH | $CH_3$ | $OCHF_2$ | |
| 365 | H | 6–F | O | $CHF_2$ | O | CH | Cl | $OCHF_2$ | |
| 366 | H | 6–F | O | $CHF_2$ | O | CH | $CH_3$ | $-O-CH_2-CF_3$ | |
| 367 | 5–$OCH_3$ | H | O | $-CH_2-CH=CH_2$ | O | N | $OCH_3$ | $-O-CH_2-CF_3$ | |
| 368 | 5–$OCH_3$ | H | O | $-CH_2-CH=CH_2$ | O | CH | $OCH_3$ | $OCHF_2$ | |
| 369 | 5–$OCH_3$ | H | O | $-CH_2-CH=CH_2$ | O | CH | $CH_3$ | $OCHF_2$ | |
| 370 | 5–$OCH_3$ | H | O | $-CH_2-CH=CH_2$ | O | CH | Cl | $OCHF_2$ | |
| 371 | 5–$OCH_3$ | H | O | $-CH_2-CH=CH_2$ | O | CH | $CH_3$ | $-O-CH_2-CF_3$ | |
| 372 | H | 3–$CH_3$ | O | $-CH_2-CH=CH_2$ | O | N | $OCH_3$ | $-O-CH_2-CF_3$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 373 | H | $3-CH_3$ | O | $-CH_2-CH=CH_2$ | O | CH | $OCH_3$ | $OCHF_2$ | |
| 374 | H | $3-CH_3$ | O | $-CH_2-CH=CH_2$ | O | CH | $CH_3$ | $OCHF_2$ | |
| 375 | H | $3-CH_3$ | O | $-CH_2-CH=CH_2$ | O | CH | Cl | $OCHF_2$ | |
| 376 | H | $3-CH_3$ | O | $-CH_2-CH=CH_2$ | O | CH | $CH_3$ | $-O-CH_2-CF_3$ | |
| 377 | H | H | O | $-CH_2-C(CH_3)Cl-CH_2-Cl$ | O | N | $OCH_3$ | $-O-CH_2-CF_3$ | |
| 378 | H | H | O | $-CH_2-C(CH_3)Cl-CH_2-Cl$ | O | CH | $OCH_3$ | $OCHF_2$ | |
| 379 | H | H | O | $-CH_2-C(CH_3)Cl-CH_2-Cl$ | O | CH | $CH_3$ | $OCHF_2$ | |
| 380 | H | H | O | $-CH_2-C(CH_3)Cl-CH_2-Cl$ | O | CH | Cl | $OCHF_2$ | |
| 381 | H | H | O | $-CH_2-C(CH_3)Cl-CH_2-Cl$ | O | CH | $CH_3$ | $-O-CH_2-CF_3$ | |
| 382 | H | H | O | $-CH_2-CHCl_2$ | O | N | $CH_3$ | $OCHF_2$ | |
| 383 | H | H | O | $-CH_2-CCl_3$ | O | N | $CH_3$ | $OCHF_2$ | |
| 384 | H | H | O | $CF_2Cl$ | O | N | $CH_3$ | $OCHF_2$ | |
| 385 | H | H | O | $CF_2Br$ | O | N | $CH_3$ | $OCHF_2$ | |
| 386 | H | H | S | $CHF_2$ | O | N | $CH_3$ | $OCHF_2$ | |
| 387 | H | H | S | $-CF_2-CHF_2$ | O | N | $CH_3$ | $OCHF_2$ | |
| 388 | H | H | $SO_2$ | $CHF_2$ | O | N | $CH_3$ | $OCHF_2$ | |
| 389 | 5−F | H | O | $CHF_2$ | O | N | $CH_3$ | $OCHF_2$ | |
| 390 | 5−F | H | O | $-CH_2-CH=CH_2$ | O | N | $CH_3$ | $OCHF_2$ | |
| 391 | H | 6−Cl | O | $CHF_2$ | O | N | $CH_3$ | $OCHF_2$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 392 | 5–Cl | H | O | $CHF_2$ | O | N | $CH_3$ | $OCHF_2$ | |
| 393 | H | 6–F | O | $CHF_2$ | O | N | $CH_3$ | $OCHF_2$ | |
| 394 | 5–$OCH_3$ | H | O | $-CH_2-CH=CH_2$ | O | N | $CH_3$ | $OCHF_2$ | |
| 395 | H | 3–$CH_3$ | O | $-CH_2-CH=CH_2$ | O | N | $CH_3$ | $OCHF_2$ | |
| 396 | H | H | O | $-CH_2-CH_2-O-C_2H_5$ | O | N | $CH_3$ | $OCHF_2$ | |
| 397 | H | H | O | $-CH_2-C=C(CH_3)_2$ | O | N | $CH_3$ | $OCHF_2$ | |
| 398 | H | H | O | $CHF_2$ | O | N | $CH_3$ | $OCHF_2$ | |
| 399 | H | H | O | $CF_3$ | O | N | $CH_3$ | $OCHF_2$ | |
| 400 | H | H | O | $-CF_2-CHF_2$ | O | N | $CH_3$ | $OCHF_2$ | |
| 401 | H | H | O | $-CH_2-CF_3$ | O | N | $CH_3$ | $OCHF_2$ | |
| 402 | H | H | O | $-CCl=CHCl$ | O | N | $CH_3$ | $OCHF_2$ | |
| 403 | H | H | O | $-CH_2-CH_2Cl$ | O | N | $CH_3$ | $OCHF_2$ | |
| 404 | H | H | O | $-CH_2-CH_2-O-CH_3$ | O | N | $CH_3$ | $OCHF_2$ | |
| 405 | H | H | O | $-CF_2-CF_3$ | O | N | $CH_3$ | $OCHF_2$ | |
| 406 | H | H | O | $-CH_2-CH=CH_2$ | O | N | $CH_3$ | $OCHF_2$ | |
| 407 | H | H | O | $-CH_2-OCH_3$ | O | N | $CH_3$ | $OCHF_2$ | |
| 408 | H | H | O | $-CF_2-CHClF$ | O | N | $CH_3$ | $OCHF_2$ | |
| 409 | H | H | O | $-CH_2-CH=CH-CH_3$ | O | N | $CH_3$ | $OCHF_2$ | |
| 410 | H | H | O | $-CH_2-CHCl-CH_2Cl$ | O | N | $CH_3$ | $OCHF_2$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 411 | H | H | O | $-CH(CH_2Cl)_2$ | O | N | $CH_3$ | $OCHF_2$ | |
| 412 | H | H | O | $-CH_2-CH_2F$ | O | N | $CH_3$ | $OCHF_2$ | |
| 413 | H | H | O | $-CH_2-C(CH_3)=CH_2$ | O | N | $CH_3$ | $OCHF_2$ | |
| 414 | H | H | O | $CHF_2$ | S | N | $OCH_3$ | $-O-CH_2-CF_3$ | |
| 415 | H | H | O | $CHF_2$ | S | CH | $CH_3$ | $OCHF_2$ | 174–175° (Zers.) |
| 416 | H | H | O | $-CH_2-CH=CH_2$ | S | N | $OCH_3$ | $-O-CH_2-CF_3$ | |
| 417 | H | H | O | $-CH_2-CH=CH_2$ | S | CH | $CH_3$ | $OCHF_2$ | |
| 418 | H | H | O | $CF_3$ | S | N | $OCH_3$ | $-O-CH_2-CF_3$ | |
| 419 | H | H | O | $CF_3$ | S | CH | $CH_3$ | $OCHF_2$ | |
| 420 | H | H | O | $-CH_2-CH_2-O-CH_3$ | S | N | $OCH_3$ | $-O-CH_2-CF_3$ | |
| 421 | H | H | O | $-CH_2-CH_2-O-CH_3$ | S | CH | $CH_3$ | $OCHF_2$ | |
| 454 | H | H | O | $CHF_2$ | O | CH | $-O-CH_2-CF_3$ | $-O-CH_2-CF_3$ | 165–166° |
| 455 | H | H | O | $CF_3$ | O | CH | $-O-CH_2-CF_3$ | $-O-CH_2-CF_3$ | |
| 456 | H | H | O | $-CF_2-CHF_2$ | O | CH | $-O-CH_2-CF_3$ | $-O-CH_2-CF_3$ | |
| 457 | H | H | O | $-CH_2-CF_3$ | O | CH | $-O-CH_2-CF_3$ | $-O-CH_2-CF_3$ | |
| 458 | H | H | O | $-CCl=CHCl$ | O | CH | $-O-CH_2-CF_3$ | $-O-CH_2-CF_3$ | |
| 459 | H | H | O | $-CH_2-CH_2Cl$ | O | CH | $-O-CH_2-CF_3$ | $-O-CH_2-CF_3$ | |
| 460 | H | H | O | $-CH_2-CH_2-O-CH_3$ | O | CH | $-O-CH_2-CF_3$ | $-O-CH_2-CF_3$ | |
| 461 | H | H | O | $-CH_2-CF_3$ | O | CH | $-O-CH_2-CF_3$ | $-O-CH_2-CF_3$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 462 | H | H | O | $-CH_2-CH=CH_2$ | O | CH | $-O-CH_2-CF_3$ | $-O-CH_2-CF_3$ | |
| 463 | H | H | O | $-CH_2-O-CH_3$ | O | CH | $-O-CH_2-CF_3$ | $-O-CH_2-CF_3$ | |
| 464 | H | H | O | $-CH_2-CHClF$ | O | CH | $-O-CH_2-CF_3$ | $-O-CH_2-CF_3$ | |
| 465 | H | H | O | $-CH_2-CH=CH-CH_3$ | O | CH | $-O-CH_2-CF_3$ | $-O-CH_2-CF_3$ | |
| 466 | H | H | O | $-CH_2-CHCl-CH_2Cl$ | O | CH | $-O-CH_2-CF_3$ | $-O-CH_2-CF_3$ | |
| 467 | H | H | O | $-CH(CH_2Cl)_2$ | O | CH | $-O-CH_2-CF_3$ | $-O-CH_2-CF_3$ | |
| 468 | H | H | O | $-CH_2-CH_2F$ | O | CH | $-O-CH_2-CF_3$ | $-O-CH_2-CF_3$ | |
| 469 | H | H | O | $-CH_2-C(CH_3)=CH_2$ | O | CH | $-O-CH_2-CF_3$ | $-O-CH_2-CF_3$ | |
| 470 | H | H | O | $-CH_2-CHCl_2$ | O | CH | $-O-CH_2-CF_3$ | $-O-CH_2-CF_3$ | |
| 471 | H | H | O | $-CH_2-CCl_3$ | O | CH | $-O-CH_2-CF_3$ | $-O-CH_2-CF_3$ | |
| 472 | H | H | O | $CF_2Cl$ | O | CH | $-O-CH_2-CF_3$ | $-O-CH_2-CF_3$ | |
| 473 | H | H | O | $CF_2Br$ | O | CH | $-O-CH_2-CF_3$ | $-O-CH_2-CF_3$ | |
| 474 | H | H | S | $CHF_2$ | O | CH | $-O-CH_2-CF_3$ | $-O-CH_2-CF_3$ | |
| 475 | H | H | S | $-CF_2-CHF_2$ | O | CH | $-O-CH_2-CF_3$ | $-O-CH_2-CF_3$ | |
| 476 | H | H | $SO_2$ | $CHF_2$ | O | CH | $-O-CH_2-CF_3$ | $-O-CH_2-CF_3$ | |
| 477 | 5–F | H | O | $CHF_2$ | O | CH | $-O-CH_2-CF_3$ | $-O-CH_2-CF_3$ | |
| 478 | 5–F | H | O | $-CH_2-CH=CH_2$ | O | CH | $-O-CH_2-CF_3$ | $-O-CH_2-CF_3$ | |
| 479 | H | 6–Cl | O | $CHF_2$ | O | CH | $-O-CH_2-CF_3$ | $-O-CH_2-CF_3$ | |
| 480 | 5–Cl | H | O | $CHF_2$ | O | CH | $-O-CH_2-CF_3$ | $-O-CH_2-CF_3$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 481 | H | 6–F | O | $CHF_2$ | O | CH | $-O-CH_2-CF_3$ | $-O-CH_2-CF_3$ | |
| 482 | 5–$OCH_3$ | H | O | $-CH_2-CH=CH_2$ | O | CH | $-O-CH_2-CF_3$ | $-O-CH_2-CF_3$ | |
| 483 | H | 3–$CH_3$ | O | $-CH_2-CH=CH_2$ | O | CH | $-O-CH_2-CF_3$ | $-O-CH_2-CF_3$ | |
| 484 | H | H | O | $-CH_2-CH_2-O-C_2H_5$ | O | CH | $-O-CH_2-CF_3$ | $-O-CH_2-CF_3$ | |
| 485 | H | H | O | $-CH_2-C=C(CH_3)_2$ | O | CH | $-O-CH_2-CF_3$ | $-O-CH_2-CF_3$ | |
| 486 | H | H | O | $CHF_2$ | O | CH | $OCHF_2$ | $OCHF_2$ | 181–182° |
| 487 | H | H | O | $CF_3$ | O | CH | $OCHF_2$ | $OCHF_2$ | 193–194° |
| 488 | H | H | O | $-CF_2-CHF_2$ | O | CH | $OCHF_2$ | $OCHF_2$ | 157–158° |
| 489 | H | H | O | $-CH_2-CF_3$ | O | CH | $OCHF_2$ | $OCHF_2$ | |
| 490 | H | H | O | $-CCl=CHCl$ | O | CH | $OCHF_2$ | $OCHF_2$ | |
| 491 | H | H | O | $-CH_2-CH_2Cl$ | O | CH | $OCHF_2$ | $OCHF_2$ | |
| 492 | H | H | O | $-CH_2-CH_2-OCH_3$ | O | CH | $OCHF_2$ | $OCHF_2$ | 137–138° |
| 493 | H | H | O | $-CF_2-CF_3$ | O | CH | $OCHF_2$ | $OCHF_2$ | 197–198° |
| 494 | H | H | O | $-CH_2-CH=CH_2$ | O | CH | $OCHF_2$ | $OCHF_2$ | |
| 495 | H | H | O | $-CH_2OCH_3$ | O | CH | $OCHF_2$ | $OCHF_2$ | |
| 496 | H | H | O | $-CF_2-CHClF$ | O | CH | $OCHF_2$ | $OCHF_2$ | |
| 497 | H | H | O | $-CH_2-CH=CH-CH_3$ | O | CH | $OCHF_2$ | $OCHF_2$ | |
| 498 | H | H | O | $-CH_2-CHCl-CH_2Cl$ | O | CH | $OCHF_2$ | $OCHF_2$ | |
| 499 | H | H | O | $-CH_2(CH_2Cl)_2$ | O | CH | $OCHF_2$ | $OCHF_2$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 500 | H | H | O | $-CH_2-CH_2F$ | O | CH | $OCHF_2$ | $OCHF_2$ | |
| 501 | H | H | O | $-CH_2-C(CH_3)=CH_2$ | O | CH | $OCHF_2$ | $OCHF_2$ | |
| 502 | H | H | O | $-CH_2-CHCl_2$ | O | CH | $OCHF_2$ | $OCHF_2$ | |
| 503 | H | H | O | $-CH_2-CCl_3$ | O | CH | $OCHF_2$ | $OCHF_2$ | |
| 504 | H | H | O | $CF_2Cl$ | O | CH | $OCHF_2$ | $OCHF_2$ | |
| 505 | H | H | O | $CF_2Br$ | O | CH | $OCHF_2$ | $OCHF_2$ | |
| 506 | H | H | S | $CHF_2$ | O | CH | $OCHF_2$ | $OCHF_2$ | 157–158° |
| 507 | H | H | S | $-CF_2-CHF_2$ | O | CH | $OCHF_2$ | $OCHF_2$ | |
| 508 | H | H | $SO_2$ | $CHF_2$ | O | CH | $OCHF_2$ | $OCHF_2$ | |
| 509 | 5–F | H | O | $CHF_2$ | O | CH | $OCHF_2$ | $OCHF_2$ | |
| 510 | 5–F | H | O | $-CH_2-CH=CH_2$ | O | CH | $OCHF_2$ | $OCHF_2$ | |
| 511 | H | 6–Cl | O | $CHF_2$ | O | CH | $OCHF_2$ | $OCHF_2$ | |
| 512 | 5–Cl | H | O | $CHF_2$ | O | CH | $OCHF_2$ | $OCHF_2$ | |
| 513 | H | 6–F | O | $CHF_2$ | O | CH | $OCHF_2$ | $OCHF_2$ | |
| 514 | $5-OCH_3$ | H | O | $-CH_2-CH=CH_2$ | O | CH | $OCHF_2$ | $OCHF_2$ | |
| 515 | H | $3-CH_3$ | O | $-CH_2-CH=CH_2$ | O | CH | $OCHF_2$ | $OCHF_2$ | |
| 516 | H | H | O | $-CH_2-CH_2-O-C_2H_5$ | O | CH | $OCHF_2$ | $OCHF_2$ | |
| 517 | H | H | O | $-CH_2-C=C(CH_3)_2$ | O | CH | $OCHF_2$ | $OCHF_2$ | |
| 518 | H | H | O | $CHF_2$ | O | N | $CH_3$ | $-O-CH_2-CH_2-Cl$ | 81° (Zers.) |

Fortsetzung

| Nr. | $R_1$ | $R_2$ | X | A | Z | E | $R_3$ | $R_4$ | Smp. [°C] |
|---|---|---|---|---|---|---|---|---|---|
| 519 | H | H | O | $CHF_2$ | O | N | $CH_3$ | $-O-CF_2-CCl_3$ | 141–142° |
| 520 | H | H | O | $CHF_2$ | O | CH | $CH_3$ | $-O-CF_2-CHFCl$ | 167–168° |
| 521 | H | H | O | $CHF_2$ | O | CH | $CH_3$ | $-O-CF_2-CHF_2$ | 151–152° |
| 522 | H | H | O | $CHF_2$ | O | CH | $CH_3$ | $-O-CF_2-CHFBr$ | |
| 523 | H | H | O | $CHF_2$ | O | CH | $OCH_3$ | $-O-CF_2-CHF_2$ | 137–138° |
| 524 | H | H | O | $CHF_2$ | O | CH | $OCH_3$ | $-O-CF_2-CHFCl$ | 125–126° |
| 525 | H | H | O | $CHF_2$ | O | CH | $OCH_3$ | $-O-CF_2-CHF-CF_3$ | |
| 526 | H | H | O | $CHF_2$ | O | CH | Cl | $-O-CF_2-CHF_2$ | |
| 527 | H | H | O | $CHF_2$ | O | CH | Cl | $-O-CF_2-CHFCl$ | 150–151° |
| 528 | H | H | O | $CHF_2$ | O | N | $CH_3$ | $-O-CF_2-CHF_2$ | |
| 529 | H | H | O | $CHF_2$ | O | N | $CH_3$ | $-O-CF_2-CHFCl$ | |
| 530 | H | H | O | $CHF_2$ | O | N | $OCH_3$ | $-O-CF_2-CHF_2$ | |
| 531 | H | H | O | $CHF_2$ | O | N | $OCH_3$ | $-O-CF_2-CF_3CHFCl$ | |
| 532 | H | H | O | $CHF_2$ | O | N | $OCH_3$ | $-O-CF_2-CHFBr$ | |
| 533 | H | H | O | $CHF_2$ | O | N | $OCH_3$ | $-O-CF_2-CHF-CF_3$ | |
| 534 | H | H | O | $CHF_2$ | O | N | $C_2H_5$ | $-O-CF_2-CHF_2$ | |
| 535 | H | H | O | $CF_3$ | O | CH | $CH_3$ | $-O-CF_2-CHFCl$ | |
| 536 | H | H | O | $CF_3$ | O | CH | $CH_3$ | $-O-CF_2-CHF_2$ | |
| 537 | H | H | O | $CF_3$ | O | CH | $CH_3$ | $-O-CF_2-CHFBr$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 538 | H | H | O | $CF_3$ | O | CH | $OCH_3$ | $-O-CF_2-CHF_2$ | |
| 539 | H | H | O | $CF_3$ | O | CH | $OCH_3$ | $-O-CF_2-CHFCl$ | |
| 540 | H | H | O | $CF_3$ | O | CH | $OCH_3$ | $-O-CF_2-CHF-CF_3$ | |
| 541 | H | H | O | $CF_3$ | O | CH | Cl | $-O-CF_2-CHF_2$ | |
| 542 | H | H | O | $CF_3$ | O | CH | Cl | $-O-CF_2-CHFCl$ | |
| 543 | H | H | O | $CF_3$ | O | N | $CH_3$ | $-O-CF_2-CHF_2$ | |
| 544 | H | H | O | $CF_3$ | O | N | $CH_3$ | $-O-CF_2-CHFCl$ | |
| 545 | H | H | O | $CF_3$ | O | N | $OCH_3$ | $-O-CF_2-CHF_2$ | |
| 546 | H | H | O | $CF_3$ | O | N | $OCH_3$ | $-O-CF_2-CHFCl$ | |
| 547 | H | H | O | $CF_3$ | O | N | $OCH_3$ | $-O-CF_2-CHFBr$ | |
| 548 | H | H | O | $CF_3$ | O | N | $OCH_3$ | $-O-CF_2-CHF-CF_3$ | |
| 549 | H | H | O | $CF_3$ | O | N | $C_2H_5$ | $-O-CF_2-CHF_2$ | |
| 550 | H | H | O | $-CH_2-CH=CH_2$ | O | CH | $CH_3$ | $-O-CF_2-CHFCl$ | |
| 551 | H | H | O | $-CH_2-CH=CH_2$ | O | CH | $CH_3$ | $-O-CF_2-CHF_2$ | |
| 552 | H | H | O | $-CH_2-CH=CH_2$ | O | CH | $CH_3$ | $-O-CF_2-CHFBr$ | |
| 553 | H | H | O | $-CH_2-CH=CH_2$ | O | CH | $OCH_3$ | $-O-CF_2-CHF_2$ | |
| 554 | H | H | O | $-CH_2-CH=CH_2$ | O | CH | $OCH_3$ | $-O-CF_2-CHFCl$ | |
| 555 | H | H | O | $-CH_2-CH=CH_2$ | O | CH | $OCH_3$ | $-O-CF_2-CHF-CF_3$ | |
| 556 | H | H | O | $-CH_2-CH=CH_2$ | O | CH | Cl | $-O-CF_2-CHF_2$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 557 | H | H | O | $-CH_2-CH=CH_2$ | O | CH | Cl | $-O-CF_2-CHFCl$ | |
| 558 | H | H | O | $-CH_2-CH=CH_2$ | O | N | $CH_3$ | $-O-CF_2-CHF_2$ | |
| 559 | H | H | O | $-CH_2-CH=CH_2$ | O | N | $CH_3$ | $-O-CF_2-CHFCl$ | |
| 560 | H | H | O | $-CH_2-CH=CH_2$ | O | N | $OCH_3$ | $-O-CF_2-CHF_2$ | |
| 561 | H | H | O | $-CH_2-CH=CH_2$ | O | N | $OCH_3$ | $-O-CF_2-CHFCl$ | |
| 562 | H | H | O | $-CH_2-CH=CH_2$ | O | N | $OCH_3$ | $-O-CF_2-CHFBr$ | |
| 563 | H | H | O | $-CH_2-CH=CH_2$ | O | N | $OCH_3$ | $-O-CF_2-CHF-CF_3$ | |
| 564 | H | H | O | $-CH_2-CH=CH_2$ | O | N | $C_2H_5$ | $-O-CF_2-CHF_2$ | |
| 565 | H | H | S | $CHF_2$ | O | CH | $CH_3$ | $-O-CF_2-CHFCl$ | |
| 566 | H | H | S | $CHF_2$ | O | CH | $CH_3$ | $-O-CF_2-CHF_2$ | |
| 567 | H | H | S | $CHF_2$ | O | CH | $CH_3$ | $-O-CF_2-CHFBr$ | |
| 568 | H | H | S | $CHF_2$ | O | CH | $CH_3$ | $-O-CF_2-CHF_2$ | |
| 569 | H | H | S | $CHF_2$ | O | CH | $OCH_3$ | $-O-CF_2-CHFCl$ | |
| 570 | H | H | S | $CHF_2$ | O | CH | $OCH_3$ | $-O-CF_2-CHF-CF_3$ | |
| 571 | H | H | S | $CHF_2$ | O | CH | Cl | $-O-CF_2-CHF_2$ | |
| 572 | H | H | S | $CHF_2$ | O | CH | Cl | $-O-CF_2-CHFCl$ | |
| 573 | H | H | S | $CHF_2$ | O | N | $CH_3$ | $-O-CF_2-CHF_2$ | |
| 574 | H | H | S | $CHF_2$ | O | N | $CH_3$ | $-O-CF_2-CHFCl$ | |
| 575 | H | H | S | $CHF_2$ | O | N | $OCH_3$ | $-O-CF_2-CHF_2$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 576 | H | H | S | $CHF_2$ | O | N | $OCH_3$ | $-O-CF_2-CHFCl$ | |
| 577 | H | H | S | $CHF_2$ | O | N | $OCH_3$ | $-O-CF_2-CHFBr$ | |
| 578 | H | H | S | $CHF_2$ | O | N | $OCH_3$ | $-O-CF_2-CHF-CF_3$ | |
| 579 | H | H | S | $CHF_2$ | O | N | $C_2H_5$ | $-O-CF_2-CHF_2$ | |
| 580 | H | H | O | $-CCl=CHCl$ | O | CH | $CH_3$ | $-O-CF_2-CHFCl$ | |
| 581 | H | H | O | $-CCl=CHCl$ | O | CH | $CH_3$ | $-O-CF_2-CHF_2$ | |
| 582 | H | H | O | $-CCl=CHCl$ | O | CH | $CH_3$ | $-O-CF_2-CHFBr$ | |
| 583 | H | H | O | $-CCl=CHCl$ | O | CH | $OCH_3$ | $-O-CF_2-CHF_2$ | |
| 584 | H | H | O | $-CCl=CHCl$ | O | CH | $OCH_3$ | $-O-CF_2-CHFCl$ | |
| 585 | H | H | O | $-CCl=CHCl$ | O | CH | $OCH_3$ | $-O-CF_2-CHF-CF_3$ | |
| 586 | H | H | O | $-CCl=CHCl$ | O | CH | Cl | $-O-CF_2-CHF_2$ | |
| 587 | H | H | O | $-CCl=CHCl$ | O | CH | Cl | $-O-CF_2-CHFCl$ | |
| 588 | H | H | O | $-CCl=CHCl$ | O | N | $CH_3$ | $-O-CF_2-CHF_2$ | |
| 589 | H | H | O | $-CCl=CHCl$ | O | N | $CH_3$ | $-O-CF_2-CHFCl$ | |
| 590 | H | H | O | $-CCl=CHCl$ | O | N | $OCH_3$ | $-O-CF_2-CHF_2$ | |
| 591 | H | H | O | $-CCl=CHCl$ | O | N | $OCH_3$ | $-O-CF_2-CHFCl$ | |
| 592 | H | H | O | $-CCl=CHCl$ | O | N | $OCH_3$ | $-O-CF_2-CHFBr$ | |
| 593 | H | H | O | $-CCl=CHCl$ | O | N | $OCH_3$ | $-O-CF_2-CHF-CF_3$ | |
| 594 | H | H | O | $-CCl=CHCl$ | O | N | $C_2H_5$ | $-O-CF_2-CHF_2$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 595 | H | H | O | $C_2H_5$ | O | CH | $CH_3$ | $-O-CF_2-CHF_2$ | |
| 596 | H | H | O | $C_2H_5$ | O | CH | $OCH_3$ | $-O-CF_2-CHF_2$ | |
| 597 | H | H | O | $C_2H_5$ | O | CH | $OCH_3$ | $-O-CF_2-CHFCl$ | |
| 598 | H | H | O | $C_2H_5$ | O | CH | $CH_3$ | $-O-CF_2-CHFCl$ | |
| 599 | H | H | O | $C_2H_5$ | O | N | $OCH_3$ | $-O-CF_2-CHFCl$ | |
| 600 | H | H | O | $C_2H_5$ | O | N | $OCH_3$ | $-O-CF_2-CF_2H$ | |
| 601 | H | H | O | $-CH_2-CH_2Cl$ | O | CH | $CH_3$ | $-O-CF_2-CHFCl$ | |
| 602 | H | H | O | $-CH_2-CH_2Cl$ | O | CH | $OCH_3$ | $-O-CF_2-CHF_2$ | |
| 603 | H | H | O | $-CH_2-CH_2Cl$ | O | N | $CH_3$ | $-O-CF_2-CHF_2$ | |
| 604 | H | H | O | $-CH_2-CH_2Cl$ | O | N | $OCH_3$ | $-O-CF_2-CHF_2$ | |
| 605 | H | H | O | $-CH_2-CH_2Cl$ | O | N | $CH_3$ | $-O-CF_2-CHFCl$ | |
| 606 | H | H | O | $-CH_2-CH_2Cl$ | O | CH | $CH_3$ | $-O-CF_2-CHF_2$ | |
| 607 | H | H | O | $-CH_2-CH_2Cl$ | O | CH | $OCH_3$ | $-O-CF_2-CHFCl$ | |
| 608 | H | H | O | $-O-(CH_2)_2-OCH_3$ | O | CH | $CH_3$ | $-O-CF_2-CHFCl$ | |
| 609 | H | H | O | $-O-(CH_2)_2-OCH_3$ | O | CH | $CH_3$ | $-O-CF_2-CHF_2$ | |
| 610 | H | H | O | $-O-(CH_2)_2-OCH_3$ | O | CH | $OCH_3$ | $-O-CF_2-CHF_2$ | |
| 611 | H | H | O | $-O-(CH_2)_2-OCH_3$ | O | CH | $OCH_3$ | $-O-CF_2-CHFCl$ | |
| 612 | H | H | O | $-O-(CH_2)_2-OCH_3$ | O | N | $CH_3$ | $-O-CF_2-CHFCl$ | |
| 613 | H | H | O | $-O-(CH_2)_2-OCH_3$ | O | N | $OCH_3$ | $-O-CF_2-CHFCl$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 614 | H | H | O | $-(CH_2)_2-S-CH_3$ | O | CH | $CH_3$ | $OCHF_2$ | 167–168° |
| 615 | H | H | O | $-(CH_2)_2-S-CH_3$ | O | CH | $OCH_3$ | $OCHF_2$ | |
| 616 | H | H | O | $-(CH_2)_2-S-CH_3$ | O | N | $OCH_3$ | $-O-CH_2-CF_3$ | |
| 617 | H | H | O | $-(CH_2)_2-S-CH_3$ | O | CH | Cl | $OCHF_2$ | |
| 618 | H | H | O | $-(CH_2)_2-S-CH_3$ | O | CH | $CH_3$ | $-O-CH_2-CF_3$ | |
| 619 | H | H | O | $-(CH_2)_2-S-CH_3$ | O | CH | $OCHF_2$ | $OCHF_2$ | |
| 620 | H | H | O | $-(CH_2)_2-S-CH_3$ | O | CH | $CH_3$ | $-O-CF_2-CHF_2$ | |
| 621 | H | H | O | $-(CH_2)_2-S-CH_3$ | O | CH | $OCH_3$ | $-O-CF_2-CHF_2$ | |
| 622 | H | H | O | $-(CH_2)_2-S-CH_3$ | O | N | $OCH_3$ | $-O-CF_2-CHF_2$ | |
| 623 | H | H | O | $-(CH_2)_2-SO-CH_3$ | O | CH | $CH_3$ | $OCHF_2$ | |
| 624 | H | H | O | $-(CH_2)_2-SO-CH_3$ | O | CH | $OCH_3$ | $OCHF_2$ | |
| 625 | H | H | O | $-(CH_2)_2-SO-CH_3$ | O | CH | Cl | $OCHF_2$ | |
| 626 | H | H | O | $-(CH_2)_2-SO-CH_3$ | O | CH | $CH_3$ | $-O-CH_2-CF_3$ | |
| 627 | H | H | O | $-(CH_2)_2-SO-CH_3$ | O | CH | $OCHF_2$ | $OCHF_2$ | |
| 628 | H | H | O | $-(CH_2)_2-SO-CH_3$ | O | CH | $CH_3$ | $-O-CF_2-CHF_2$ | |
| 629 | H | H | O | $-(CH_2)_2-SO-CH_3$ | O | CH | $OCH_3$ | $-O-CF_2-CHF_2$ | |
| 630 | H | H | O | $-(CH_2)_2-SO-CH_3$ | O | N | $OCH_3$ | $-O-CH_2-CF_3$ | |
| 631 | H | H | O | $-(CH_2)_2-SO-CH_3$ | O | CH | $OCH_3$ | $-O-CF_2-CHF_2$ | |
| 632 | H | H | O | $-(CH_2)_2-SO_2-CH_3$ | O | CH | $CH_3$ | $OCHF_2$ | |

Fortsetzung

| Nr. | $R_1$ | $R_2$ | X | A | Z | E | $R_3$ | $R_4$ | Smp. [°C] |
|---|---|---|---|---|---|---|---|---|---|
| 633 | H | H | O | $-(CH_2)_2-SO_2-CH_3$ | O | CH | $OCH_3$ | $OCHF_2$ | |
| 634 | H | H | O | $-(CH_2)_2-SO_2-CH_3$ | O | CH | Cl | $OCHF_2$ | |
| 635 | H | H | O | $-(CH_2)_2-SO_2-CH_3$ | O | CH | $CH_3$ | $-O-CH_2-CF_3$ | |
| 636 | H | H | O | $-(CH_2)_2-SO_2-CH_3$ | O | CH | $OCHF_2$ | $OCHF_2$ | |
| 637 | H | H | O | $-(CH_2)_2-SO_2-CH_3$ | O | CH | $CH_3$ | $-O-CF_2-CHF_2$ | |
| 638 | H | H | O | $-(CH_2)_2-SO_2-CH_3$ | O | CH | $OCH_3$ | $-O-CF_2-CHF_2$ | |
| 639 | H | H | O | $-(CH_2)_2-SO_2-CH_3$ | O | N | $OCH_3$ | $-O-CH_2-CF_3$ | |
| 640 | H | H | O | $-(CH_2)_2-SO_2-CH_3$ | O | CH | $OCH_3$ | $-O-CF_2-CHF_2$ | |

Tabelle 2

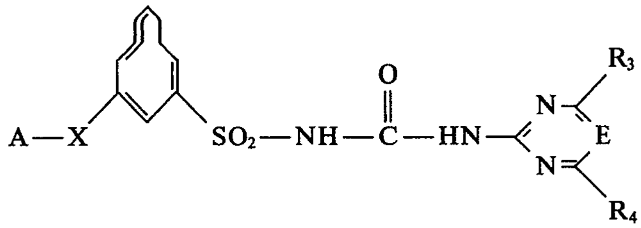

| Nr. | X | A | E | $R_3$ | $R_4$ | Smp. [°C] |
|---|---|---|---|---|---|---|
| 701 | O | $CHF_2$ | CH | $OCH_3$ | $OCHF_2$ | 148–149° |
| 702 | O | $CHF_2$ | CH | $CH_3$ | $OCHF_2$ | |
| 703 | O | $CHF_2$ | CH | Cl | $OCHF_2$ | |
| 704 | O | $CHF_2$ | CH | $CH_3$ | $-O-CH_2-CF_3$ | |
| 705 | O | $CHF_2$ | N | $OCH_3$ | $-O-CH_2-CF_3$ | |
| 706 | O | $-CF_2-CHF_2$ | CH | $OCH_3$ | $OCHF_2$ | |
| 707 | O | $-CF_2-CHF_2$ | CH | $CH_3$ | $OCHF_2$ | |
| 708 | O | $-CF_2-CHF_2$ | CH | Cl | $OCHF_2$ | |
| 709 | O | $-CF_2-CHF_2$ | CH | $CH_3$ | $-O-CH_2-CF_3$ | |
| 710 | O | $-CF_2-CHF_2$ | N | $OCH_3$ | $-O-CH_2-CF_3$ | |

## Formulierungsbeispiele

### Beispiel 8

Formulierungsbeispiele für Wirkstoffe der Formel I (%= Gewichtsprozent)

a) Spritzpulver

| | a) | b) | c) |
|---|---|---|---|
| Wirkstoff | 20% | 60% | 0,5% |
| Na-Ligninsulfonat | 5% | 5% | 5% |
| Na-Laurylsulfat | 3% | – | – |
| Na-Diisobutylnaphthalinsulfonat | – | 6% | 6% |
| Octylphenolpolyäthylenglykoläther (7–8 Mol AeO) | – | 2% | 2% |
| Hochdisperse Kieselsäure | 5% | 27% | 27% |
| Kaolin | 67% | – | – |
| Natriumchlorid | – | – | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsion-Konzentrat

| | a) | b) |
|---|---|---|
| Wirkstoff | 10% | 1% |
| Octylphenolpolyäthylenglykoläther (4–5 Mol AeO) | 3% | 3% |
| Ca-Dodecylbenzolsulfat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff | 0,1% | 1% |
| Talkum | 99,9% | – |
| Kaolin | – | 99% |

Man erhält anwendungsfertige Stäubemittel, indem der Wirkstoff mit dem Träger vermischt und auf einer geeigneten Mühle vermahlen wird.

d) Extruder Granulat

| | a) | b) |
|---|---|---|
| Wirkstoff | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschließend im Luftstrom getrocknet.

e) Umhüllungs-Granulat

| | |
|---|---|
| Wirkstoff | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmäßig aufgetragen. Auf diese Weise erhält man staubfreie Umhüllungs-Granulate.

f) Suspensions-Konzentrat

| | a) | b) |
|---|---|---|
| Wirkstoff | 40% | 5% |
| Äthylenglykol | 10% | 10% |
| Nonylphenolpolyäthylenglykoläther (15 Mol AeO) | 6% | 1% |
| Na-Ligninsulfonat | 10% | 5% |
| Carboxymethylcellulose | 1% | 1% |
| 37%ige wäßrige Formaldehyd-Lösung | 0,2% | 0,2% |
| Silikonöl in Form einer 75%igen wäßrigen Emulsion | 0,8% | 0,8% |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff | 5% |
| Isopropylamin | 1% |
| Octylphenolpolyäthylenglykoläther (78 Mol AeO) | 3% |
| Wasser | 91% |

## Biologische Beispiele

### Beispiel 9

#### Nachweis der Herbizidwirkung vor dem Auflaufen der Pflanzen

Im Gewächshaus werden Pflanzensamen in Blumentöpfe von 12–15 cm Durchmesser gesät. Unmittelbar danach wird die Erdoberfläche mit einer wäßrigen Dispersion oder Lösung der Wirkstoffe behandelt. Es werden Konzentrationen von 4 kg Wirkstoffmengen pro Hektar angewendet. Die Töpfe werden dann im Gewächshaus bei einer Temperatur von 22–25°C und 50–70% relativer Luftfeuchtigkeit gehalten. Nach 3 Wochen wird der Versuch ausgewertet und die Wirkung auf die Versuchspflanzen nach dem folgenden Maßstab bewertet:

1: Pflanze nicht gekeimt oder total abgestorben
2–3: sehr starke Wirkung
4–6: mittlere Wirkung
7–9: schwache Wirkung
9: keine Wirkung (wie unbehandelte Kontrolle).

Pre-emergente Wirkung

Aufwandmenge: 4 kg Wirksubstanz/Hektar

| Verb. Nr. | Avena | Setaria | Sinapis | Stellaria |
|---|---|---|---|---|
| 2 | 4 | 3 | 2 | 2 |
| 31 | 2 | 1 | 2 | 2 |

### Beispiel 10

#### Nachweis der Selektivität bei Vorauflaufanwendung

In der gleichen Versuchsanordnung wie im Beispiel 9 werden eine größere Anzahl von Pflanzensamen mit verschiedenen Aufwandmengen an Wirksubstanz behandelt. Die Auswertung erfolgt nach dem gleichen Maßstab.

Pre-emergente Wirkung

| Wirkung | Verb. Nr. 1 | | Verb. Nr. 6 | | Verb. Nr. 25 | |
|---|---|---|---|---|---|---|
| Aufwandmenge kg AS/ha Testpflanze | 0,06 | 0,03 | 0,06 | 0,03 | 0,06 | 0,03 |
| Weizen | 9 | 9 | 7 | 9 | 9 | 9 |
| Mais | 2 | 6 | 6 | 7 | 9 | 9 |
| Alopecurus myos. | 2 | 2 | 4 | 6 | 6 | 6 |
| Cyperus escul. | 5 | 6 | 2 | 2 | 4 | 4 |
| Abutilon | 3 | 3 | 2 | 3 | 3 | 3 |
| Chenopodium Sp. | 3 | 3 | 2 | 2 | 3 | 3 |
| Ipomoea | 4 | 8 | 2 | 2 | 8 | 9 |

Fortsetzung

| Wirkung | Verb. Nr. 1 | | Verb. Nr. 6 | | Verb. Nr. 25 | |
|---|---|---|---|---|---|---|
| Aufwandmenge kg AS/ha Testpflanze | 0,06 | 0,03 | 0,06 | 0,03 | 0,06 | 0,03 |
| Sinapis | 2 | 2 | 2 | 2 | 2 | 2 |
| Galium | 4 | 4 | 2 | 2 | 2 | 3 |
| Viola tricolor | 2 | 2 | 1 | 1 | 2 | 3 |

Beispiel 11

Nachweis der Herbizidwirkung nach dem Auflaufen der Pflanzen (Kontaktwirkung)

Eine Anzahl Unkräuter und Kulturpflanzen, sowohl monokotyle wie dikotyle, werden nach dem Auflaufen, im 4- bis 6-Blattstadium mit einer wäßrigen Wirkstoffdispersion in Dosierung von 4 kg AS/ha gespritzt und dann bei 24° bis 26 °C und 45—60% relativer Luftfeuchtigkeit gehalten. 15 Tage nach der Behandlung wird der Versuch ausgewertet und nach dem gleichen Maßstab wie im pre-emergenten Versuch bewertet.

Post-emergente Wirkung

Aufwandmenge: 4 kg Wirksubstanz/Hektar

| Verb. Nr. | Avena | Setaria | Lolium | Solanum | Sinapis | Stellaria | Phaseolus |
|---|---|---|---|---|---|---|---|
| 2 | 5 | 2 | 2 | 2 | 2 | 2 | 3 |
| 31 | 2 | 3 | 3 | 2 | 2 | 3 | 3 |

Beispiel 12

Nachweis der Selektivität bei Nachauflaufanwendung

In der gleichen Versuchsanordnung wie im Beispiel 11 werden eine größere Anzahl von Pflanzen mit verschiedenen Aufwandmengen an Wirksubstanz behandelt. Die Auswertung erfolgt nach dem in Beispiel 9 angegebenen Maßstab.

Post-emergente Wirkung

| Wirkung | Verb. Nr. 6 | | Verb. Nr. 25 | |
|---|---|---|---|---|
| Aufwandmenge kg AS/ha Testpflanze | 0,06 | 0,03 | 0,06 | 0,03 |
| Weizen | 8 | 9 | 9 | 9 |
| Mais | 9 | 9 | 9 | 9 |
| Reis trocken | 7 | 9 | 9 | 9 |
| Cyperus escul. | 3 | 4 | 4 | 4 |
| Baumwolle | 9 | 9 | 9 | 9 |

Fortsetzung

| Wirkung | Verb. Nr. 6 | | Verb. Nr. 25 | |
|---|---|---|---|---|
| Aufwandmenge kg AS/ha Testpflanze | 0,06 | 0,03 | 0,06 | 0,03 |
| Xanthium Sp. | 2 | 2 | 1 | 1 |
| Chenopodium Sp. | 3 | 3 | 9 | 9 |
| Sinapis | 2 | 3 | 3 | 4 |
| Galium aparine | 1 | 2 | 6 | 7 |
| Viola tricolor | 3 | 4 | 3 | 4 |

Beispiel 13

Nachweis der Keimhemmung an Lagerkartoffeln

Eine Anzahl im Handel erhältliche Kartoffeln der Sorte »Urgenta« ohne Keime werden gewaschen und abgetrocknet. Danach werden die Kartoffeln für jeweils eine Minute in Wirkstoffemulsionen verschiedener Konzentration getaucht, in Kunststoffschalen auf Filterpapier ausgelegt und bei Temperaturen von 14° und 21 °C im Dunkeln bei 50% relativer Luftfeuchtigkeit gehalten. Die Auswertung erfolgte 34 Tage nach der Applikation. Gleichzeitig wird der Gewichtsverlust der Knollen und das Gewicht der Keime im Vergleich zur unbehandelten Kontrolle ermittelt. Die erfindungsgemäßen Verbindungen zeigten in diesem Versuch eine vollständige Verhinderung der Keimbildung. Gleichzeitig betrug der Gewichtsverlust der Kartoffeln weniger als 10% des Gewichtsverlustes der Kontrollkartoffeln.

Beispiel 14

Nachweis der Wuchshemmung bei tropischen Bodendeckern-Leguminosen (cover crops)

In Kunststoffschalen mit Erde-Torf-Sand-Gemisch (1:1:1) werden Testpflanzen der Sorte Psophocarpus palustris und Centrosema pubescens aus Stecklingen angezogen. Nach dem Anwurzeln werden die Pflänzchen in 9-cm-Töpfe umgetopft und nach Bedarf gewässert. Die weitere Anzucht der Pflanzen findet im Gewächshaus bei einer Tagestemperatur von 27°C und einer Nachttemperatur von 21°C, bei einer mittleren Lichtdauer von 14 h (6000 Lux) und einer Luftfeuchtigkeit von 70% statt. Die Testpflanzen werden auf eine Höhe von ca. 15 cm zurückgeschnitten und 7 Tage nach dem Zurückschneiden mit einer Spritzbrühe des Wirkstoffs (umgerechnet 0,3 bzw. 3 kg) Aktivsubstanz pro Hektar) besprüht. 4 Wochen nach Applikation wird das Wachstum der behandelten Pflanzen im Vergleich zu gestutzten, aber unbehandelten Kontrollpflanzen verglichen. In diesem Versuch zeigen die mit den Wirkstoffen der Formel I behandelten Pflanzen eine deutliche Reduktion des Neuzuwachses (weniger als 20%des Neuzuwachses bei unbehandelten Kontrollpflanzen), ohne daß dabei die Versuchspflanzen geschädigt werden.

Beispiel 15

Nachweis der pre-emergenten Selektivität in Soja-Kulturen

In Kunststoffbehältern mit 30 l Inhalt (60×30×25 cm) werden in sandig-lehmiger Ackererde die Versuchspflanzen ausgesät, mit einer 1–2 cm dicken Erdeschicht abgedeckt und mit Wasser angegossen. Nach einem Tag wird der Wirkstoff in Form einer wäßrigen Spritzbrühe in einer Menge entsprechend 500 l/ha in Konzentrationen entsprechend 60 g/ha und 30 g/ha appliziert. Anschließend werden die Behälter im Gewächshaus bei Temperaturen von 20–25°C gehalten und bei Bedarf gegossen. Nach 33 Tagen wird der Versuch ausgewertet und nach dem gleichen Maßstab wie im Beispiel 9 bonitiert.

Pre-emergente Wirkung in Soja-Kulturen

Wirkstoff Nr. 31

| Versuchspflanze | Aufwandmenge | |
|---|---|---|
| | 0,06 kg/ha | 0,03 kg/ha |
| Soja | 7 | 8 |
| Amaranthus ret. | 2 | 3 |
| Ipomoea p. | 3 | 4 |
| Portulaca o. | 1 | 2 |
| Brachiaria pl. | 3 | 4 |
| Xanthium can. | 3 | 3 |

## Beispiel 16

### Wuchsregulierung an Sojabohnen

In Kunststoffbehältern mit einem Erde-Torf-Sandgemisch im Verhältnis 6:3:1 werden Sojabohnen der Sorte ›Hark‹ angesät und in eine Klimakammer gegeben. Durch optimale Temperaturwahl, Beleuchtung, Düngerzugabe und Bewässerung entwickeln sich die Pflanzen nach ca. 5 Wochen bis zum 5–6 Trifolia-Blattstadium. Zu diesem Zeitpunkt werden die Pflanzen mit der wäßrigen Brühe eines Wirkstoffes der Formel I bis zur guten Benetzung besprüht. Die Wirkstoffkonzentration beträgt bis zu 100 g AS/ha. Die Auswertung erfolgt ca. 5 Wochen nach der Applikation des Wirkstoffs. Im Vergleich zu unbehandelten Kontrollpflanzen bewirken die erfindungsgemäßen Wirkstoffe der Formel I eine merkliche Erhöhung der Anzahl und des Gewichts der Schoten am Haupttrieb.

Wuchsregulierende Wirkung in Soja-Kulturen

Wirkstoff Nr. 31

| Aufwandmenge g/ha | Höhe der Pflanzen in % zur Kontrolle | Anzahl Schoten am Haupttrieb in % zur Kontrolle | Gewicht Schoten am Haupttrieb in % zur Kontrolle |
|---|---|---|---|
| 3 | 100 | 130 | 124 |
| 10 | 99 | 115 | 106 |
| 30 | 99 | 110 | 100 |
| 100 | 89 | 115 | 112 |

**Patentansprüche für die Vertragsstaaten: BE, CH, DE, FR, GB, IT, L I, NL, SE**

1. N-Phenylsulfonyl-N'-pyrimidinyl- und -triazinyl-harnstoffe der allgemeinen Formel I

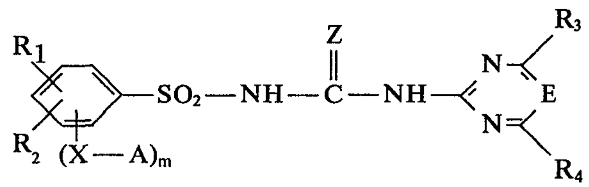

(I)

worin

A einen durch Halogen, $C_1$—$C_4$-Alkoxy, $C_1$—$C_4$-Alkylthio, $C_1$—$C_4$-Alkylsulfinyl, $C_1$—$C_4$-Alkylsulfonyl, $C_1$—$C_4$-Halogenalkoxy, $C_1$—$C_4$-Halogenalkylthio, $C_1$—$C_4$-Halogenalkylsulfinyl oder $C_1$—$C_4$-Halogenalkylsulfonyl substituierten $C_1$—$C_6$-Alkylrest oder einen durch die aufgezählten Reste substituierten $C_2$—$C_6$-Alkenylrest,
E die Methingruppe oder Stickstoff,
X Sauerstoff, Schwefel, eine Sulfinyl- oder Sulfonylbrücke,
Z Sauerstoff oder Schwefel,
m die Zahl eins oder zwei,
$R_1$ Wasserstoff, Halogen, $C_1$—$C_5$-Alkyl, $C_2$—$C_5$-Alkenyl oder einen Rest —Y—$R_5$,
$R_2$ Wasserstoff, Halogen, $C_1$—$C_5$-Alkyl, $C_2$—$C_5$-Alkenyl, $C_1$—$C_4$-Halogenalkyl, oder einen Rest —Y—$R_5$, —$COOR_6$, —$NO_2$ oder —CO—$NR_7$—$R_8$,
$R_3$ Wasserstoff, $C_1$—$C_4$-Alkyl, $C_1$—$C_4$-Alkoxy, $C_1$—$C_4$-Alkylthio, $C_1$—$C_4$-Halogenalkyl, Halogen, $C_1$—$C_4$-Halogenalkoxy oder Alkoxyalkyl mit höchstens 4 Kohlenstoffatomen,
$R_4$ $C_1$—$C_4$-Halogenalkoxy,
$R_5$ und $R_6$ je $C_1$—$C_5$-Alkyl, $C_2$—$C_5$-Alkenyl oder $C_2$—$C_6$-Alkinyl,
$R_7$ und $R_8$ unabhängig voneinander Wasserstoff, $C_1$—$C_5$-Alkyl, $C_2$—$C_5$-Alkenyl oder $C_2$—$C_6$-Alkinyl und
Y Sauerstoff, Schwefel, eine Sulfinyl- oder Sulfonylbrücke bedeuten,

wobei A auch für den unsubstituierten $C_2$—$C_6$-Alkenylrest stehen kann, wenn gleichzeitig X Sauerstoff bedeutet, sowie die Salze dieser Verbindungen.

2. Verbindungen gemäß Anspruch 1, dadurch gekennzeichnet, daß

A einen durch Halogen, $C_1$—$C_4$-Alkoxy, $C_1$—$C_4$-Alkylthio, $C_1$—$C_4$-Alkylsulfinyl, $C_1$—$C_4$-Alkylsulfonyl, $C_1$—$C_4$-Halogenalkoxy, $C_1$—$C_4$-Halogenalkylthio, $C_1$—$C_4$-Halogenalkylsulfinyl oder $C_1$—$C_4$-Halogenalkylsulfonyl substituierten $C_1$—$C_6$-Alkylrest oder einen durch die aufgezählten Reste substituierten $C_2$—$C_6$-Alkenylrest,
E die Methingruppe oder Stickstoff,
X Sauerstoff, Schwefel, eine Sulfinyl- oder Sulfonylbrücke,
Z Sauerstoff oder Schwefel,
m die Zahl eins oder zwei,
$R_1$ Wasserstoff, Halogen, $C_1$—$C_5$-Alkyl, $C_2$—$C_5$-Alkenyl oder einen Rest —Y—$R_5$,
$R_2$ Wasserstoff, Halogen, $C_1$—$C_5$-Alkyl, $C_2$—$C_5$-Alkenyl, $C_1$—$C_4$-Halogenalkyl, oder einen Rest —Y—$R_5$, —$COOR_6$, —$NO_2$ oder —CO—$NR_7$—$R_8$,
$R_3$ Wasserstoff, $C_1$—$C_4$-Alkyl, $C_1$—$C_4$-Alkoxy, $C_1$—$C_4$-Alkylthio, $C_1$—$C_4$-Halogenalkyl, Halogen oder Alkoxyalkyl mit höchstens 4 Kohlenstoffatomen,
$R_4$ $C_1$—$C_4$-Halogenalkoxy,
$R_5$ und $R_6$ je $C_1$—$C_5$-Alkyl, $C_2$—$C_5$-Alkenyl oder $C_2$—$C_6$-Alkinyl,
$R_7$ und $R_8$ unabhängig voneinander Wasserstoff, $C_1$—$C_5$-Alkyl, $C_2$—$C_5$-Alkenyl oder $C_2$—$C_6$-Alkinyl und
Y Sauerstoff, Schwefel, eine Sulfinyl- oder Sulfonylbrücke bedeuten,

wobei A auch für den unsubstituierten $C_2$—$C_6$-Alkenylrest stehen kann, wenn gleichzeitig X Sauerstoff bedeutet, sowie die Salze dieser Verbindungen.

3. Verbindungen gemäß Anspruch 1, dadurch gekennzeichnet, daß Z Sauerstoff bedeutet.

4. Verbindungen gemäß Anspruch 1, dadurch gekennzeichnet, daß X Sauerstoff bedeutet.

5. Verbindungen gemäß Anspruch 1, dadurch gekennzeichnet, daß die Reste $R_3$ und $R_4$ zusammen nicht mehr als 4 Kohlenstoffatome enthalten.

6. Verbindungen gemäß Anspruch 1, dadurch gekennzeichnet, daß m die Zahl eins bedeutet.

7. Verbindungen gemäß Anspruch 1, dadurch gekennzeichnet, daß X und Z Sauerstoff und m die Zahl eins bedeuten und der Rest —X—A in der 2-Position zur Sulfonylgruppe steht.

8. Verbindungen gemäß Anspruch 7, dadurch gekennzeichnet, daß die Reste $R_3$ und $R_4$ zusammen nicht mehr als 4 Kohlenstoffatome enthalten.

9. Verbindungen gemäß Anspruch 8, dadurch gekennzeichnet, daß $R_4$ für Halogenäthoxy steht.

10. Verbindungen gemäß Anspruch 8, dadurch gekennzeichnet, daß $R_4$ für Halogenmethoxy steht.

11. Verbindungen gemäß Anspruch 9, dadurch gekennzeichnet, daß $R_4$ den 2,2,2-Trifluoräthoxyrest bedeutet.

12. Verbindungen gemäß Anspruch 10, dadurch gekennzeichnet, daß $R_4$ den Difluormethoxyrest bedeutet.

13. N-(2-Difluormethoxyphenyl-sulfonyl)-N'-[4-methoxy-6-(2,2,2-trifluoräthoxy)-1,3,5-triazin-2-yl]-harnstoff gemäß Anspruch 1.

14. N-(2-Difluormethoxyphenyl-sulfonyl)-N'-(4-difluormethoxy-6-methyl-pyrimidin-2-yl)-harnstoff gemäß Anspruch 1.

15. Verfahren zur Herstellung der Verbindungen der Formel I, Anspruch 1, dadurch gekennzeichnet, daß man ein Phenylsulfonamid der Formel II

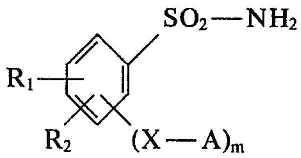

(II)

worin A, $R_1$, $R_2$, X und m die unter Formel I gegebenen Bedeutung haben, in Gegenwart einer Base mit einem N-Pyrimidinyl- oder -Triazinylcarbamat der Formel III

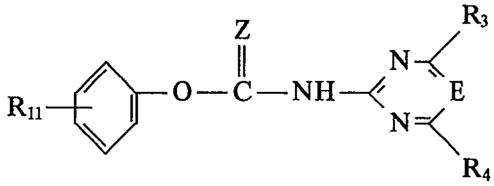

(III)

worin E, $R_3$, $R_4$ und Z die unter Formel I gegebene Bedeutung haben, und $R_{11}$ für Wasserstoff, Halogen, Nitro oder $C_1$—$C_3$-Alkyl steht, umsetzt und gegebenenfalls in ihre Salze überführt.

16. Verfahren zur Herstellung der Verbindungen der Formel I, Anspruch 1, dadurch gekennzeichnet, daß man ein Phenylsulfonylisocyanat oder -isothiocyanat der Formel IV

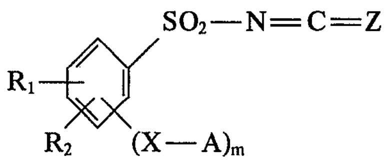

(IV)

worin A, $R_1$, $R_2$, m, X und Z die unter Formel I gegebene Bedeutung haben, gegebenenfalls in Gegenwart einer Base, mit einem Amin der Formel V

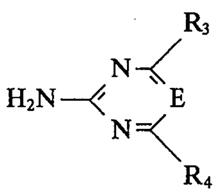

(V)

worin E, $R_3$ und $R_4$ die unter Formel I gegebene Bedeutung haben, umsetzt und gegebenenfalls in ihre Salze überführt.

17. Verfahren zur Herstellung der Verbindungen der Formel I, Anspruch 1, dadurch gekennzeichnet, daß man Sulfonamide der oben angegebenen Formel II gegebenenfalls in Gegenwart einer Base mit einem Isocyanat der Formel VI

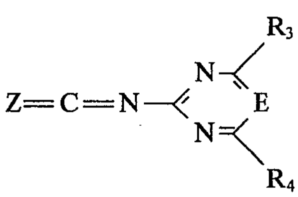

(VI)

worin E, $R_3$, $R_4$ und Z die unter Formel I gegebene Bedeutung haben, umsetzt und gegebenenfalls in ihre Salze überführt.

18. Verfahren zur Herstellung der Verbindungen der Formel I, Anspruch 1, dadurch gekennzeichnet, daß man ein N-Phenylsulfonylcarbamat der Formel VII

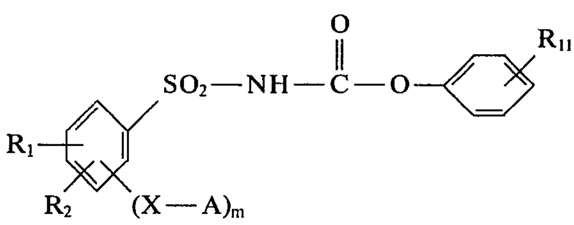

(VII)

worin A, $R_1$, $R_2$, m und X die unter Formel I gegebene Bedeutung haben und $R_{11}$ für Wasserstoff, Halogen, Nitro oder $C_1$—$C_3$-Alkyl steht, mit einem Amin der oben angegebenen Formel V umsetzt und gegebenenfalls in ihre Salze überführt.

19. Verfahren zur Herstellung von Additionssalzen der Formel I gemäß einem der Ansprüche 15 bis 18, dadurch gekennzeichnet, daß man einen Sulfonylharnstoff der Formel I mit einem Amin, einem Alkalimetall- oder Erdalkalimetallhydroxid oder einer quaternären Ammoniumbase umsetzt.

20. Ein herbizides und den Pflanzenwuchs hemmendes Mittel, dadurch gekennzeichnet, daß es neben Träger- und/oder anderen Zuschlagstoffen als Wirkstoff mindestens einen N-Phenylsulfonyl-N'-triazinyl- oder -pyrimidinyl-harnstoff der Formel I, Anspruch 1, enthält.

21. Die Verwendung der N-Phenylsulfonyl-N'-triazinyl- oder -pyrimidinyl-harnstoffe der Formel I, Anspruch 1, oder sie enthaltender Mittel zur Bekämpfung unerwünschten Pflanzenwachstums.

22. Die Verwendung der N-Phenylsulfonyl-N'-triazinyl- oder -pyrimidinyl-harnstoffe der Formel I, Anspruch 1, oder sie enthaltender Mittel zur Hemmung des Pflanzenwachstums.

23. Die Verwendung gemäß Anspruch 21 zur selektiven pre- oder postemergenten Bekämpfung von Unkräutern in Nutpflanzenkulturen.

24. Die Verwendung gemäß Anspruch 23 in Getreide-, Mais-, Reis- und Baumwollkulturen.

25. Die Verwendung gemäß Anspruch 23 in Sojakulturen.

26. Die Verwendung gemäß Anspruch 22, zur Unterdrückung des Pflanzenwachstums über das 2-Blattstadium hinaus, dadurch gekennzeichnet, daß die Wirkstoffe pre-emergent angewendet werden.

27. Die Verwendung der N-Phenylsulfonyl-N'-triazinyl- oder -pyrimidinyl-harnstoffe der Formel I, Anspruch 1, oder sie enthaltender Mittel zur Wuchsregulierung von Kulturpflanzen zum Zwecke einer Ertragssteigerung.

28. Die Verwendung gemäß Anspruch 27 in Sojakulturen.

29. Die Verwendung gemäß Anspruch 22 bei Bodenbedecker-Leguminosen.

30. N-[2-(2-Chloräthoxy)-phenyl-sulfonyl]-N'-[4-methoxy-6-(2,2,2-trifluoräthoxy)-1,3,5-triazin-2-yl]-harnstoff, gemäß Anspruch 1.

31. N-(2-Trifluormethoxyphenyl-sulfonyl)-N'-(4-difluormethoxy-6-methyl-pyrimidin-2-yl)-harnstoff gemäß Anspruch 1.

**Patentansprüche für den Vertragsstaat: AT**

1. Ein herbizides und den Pflanzenwuchs hemmendes Mittel, dadurch gekennzeichnet, daß es neben Träger- und/oder anderen Zuschlagstoffen als Wirkstoff mindestens einen N-Phenylsulfonyl-N'-triazinyl- oder -pyrimidinyl-harnstoff der Formel I

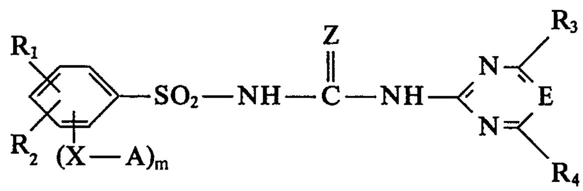

(I)

oder ein Salz davon enthält, worin

A einen durch Halogen, $C_1$—$C_4$-Alkoxy, $C_1$—$C_4$-Alkylthio, $C_1$—$C_4$-Alkylsulfinyl, $C_1$—$C_4$-Alkylsulfonyl, $C_1$—$C_4$-Halogenalkoxy, $C_1$—$C_4$-Halogenalkylthio, $C_1$—$C_4$-Halogenalkylsulfinyl oder $C_1$—$C_4$-Halogenalkylsulfonyl substituierten $C_1$—$C_6$-Alkylrest oder einen durch die aufgezählten Reste substituierten $C_2$—$C_6$-Alkenylrest,
E die Methingruppe oder Stickstoff,
X Sauerstoff, Schwefel, eine Sulfinyl- oder Sulfonylbrücke,
Z Sauerstoff oder Schwefel,
m die Zahl eins oder zwei,
$R_1$ Wasserstoff, Halogen, $C_1$—$C_5$-Alkyl, $C_2$—$C_5$-Alkenyl oder einen Rest —Y—$R_5$,
$R_2$ Wasserstoff, Halogen, $C_1$—$C_5$-Alkyl, $C_2$—$C_5$-Alkenyl, $C_1$—$C_4$-Halogenalkyl, oder einen Rest —Y—$R_5$, —$COOR_6$, —$NO_2$ oder —CO—$NR_7$—$R_8$,
$R_3$ Wasserstoff, $C_1$—$C_4$-Alkyl, $C_1$—$C_4$-Alkoxy, $C_1$—$C_4$-Alkylthio, $C_1$—$C_4$-Halogenalkyl, Halogen, $C_1$—$C_4$-Halogenalkoxy oder Alkoxyalkyl mit höchstens 4 Kohlenstoffatomen,
$R_4$ $C_1$—$C_4$-Halogenalkoxy,
$R_5$ und $R_6$ je $C_1$—$C_5$-Alkyl, $C_2$—$C_5$-Alkenyl oder $C_2$—$C_6$-Alkinyl,
$R_7$ und $R_8$ unabhängig voneinander Wasserstoff, $C_1$—$C_5$-Alkyl, $C_2$—$C_5$-Alkenyl oder $C_2$—$C_6$-Alkinyl und
Y Sauerstoff, Schwefel, eine Sulfinyl- oder Sulfonylbrücke bedeuten,

wobei A auch für den unsubstituierten $C_2$—$C_6$-Alkenylrest stehen kann, wenn gleichzeitig X Sauerstoff bedeutet.

2. Mittel gemäß Anspruch 1, dadurch gekennzeichnet, daß

A einen durch Halogen, $C_1$—$C_4$-Alkoxy, $C_1$—$C_4$-Alkylthio, $C_1$—$C_4$-Alkylsulfinyl, $C_1$—$C_4$-Alkylsulfonyl, $C_1$—$C_4$-Halogenalkoxy, $C_1$—$C_4$-Halogenalkylthio, $C_1$—$C_4$-Halogenalkylsulfinyl oder $C_1$—$C_4$-Halogenalkylsulfonyl substituierten $C_1$—$C_6$-Alkylrest oder einen durch die aufgezählten Reste substituierten $C_2$—$C_6$-Alkenylrest,
E die Methingruppe oder Stickstoff,
X Sauerstoff, Schwefel, eine Sulfinyl- oder Sulfonylbrücke,
Z Sauerstoff oder Schwefel,
m die Zahl eins oder zwei,
$R_1$ Wasserstoff, Halogen, $C_1$—$C_5$-Alkyl, $C_2$—$C_5$-Alkenyl oder einen Rest —Y—$R_5$,
$R_2$ Wasserstoff, Halogen, $C_1$—$C_5$-Alkyl, $C_2$—$C_5$-Alkenyl, $C_1$—$C_4$-Halogenalkyl, oder einen Rest —Y—$R_5$, —$COOR_6$, —$NO_2$ oder —CO—$NR_7$—$R_8$,
$R_3$ Wasserstoff, $C_1$—$C_4$-Alkyl, $C_1$—$C_4$-Alkoxy, $C_1$—$C_4$-Alkylthio, $C_1$—$C_4$-Halogenalkyl, Halogen, oder Alkoxyalkyl mit höchstens 4 Kohlenstoffatomen,
$R_4$ $C_1$—$C_4$-Halogenalkoxy,
$R_5$ und $R_6$ je $C_1$—$C_5$-Alkyl, $C_2$—$C_5$-Alkenyl oder $C_2$—$C_6$-Alkinyl,
$R_7$ und $R_8$ unabhängig voneinander Wasserstoff, $C_1$—$C_5$-Alkyl, $C_2$—$C_5$-Alkenyl oder $C_2$—$C_6$-Alkinyl und
Y Sauerstoff, Schwefel, eine Sulfinyl- oder Sulfonylbrücke bedeuten,

wobei A auch für den unsubstituierten $C_2$—$C_6$-Alkenylrest stehen kann, wenn gleichzeitig X Sauerstoff bedeutet.

3. Mittel gemäß Anspruch 1, dadurch gekennzeichnet, daß Z Sauerstoff bedeutet.

4. Mittel gemäß Anspruch 1, dadurch gekennzeichnet, daß X Sauerstoff bedeutet.

5. Mittel gemäß Anspruch 1, dadurch gekennzeichnet, daß die Reste $R_3$ und $R_4$ zusammen nicht mehr als 4 Kohlenstoffatome enthalten.

6. Mittel gemäß Anspruch 1, dadurch gekennzeichnet, daß m die Zahl eins bedeutet.

7. Mittel gemäß Anspruch 1, dadurch gekennzeichnet, daß X und Z Sauerstoff und m die Zahl eins bedeuten und der Rest —X—A in der 2-Position zur Sulfonylgruppe steht.

8. Mittel gemäß Anspruch 1, dadurch gekennzeichnet, daß die Reste $R_3$ und $R_4$ zusammen nicht mehr als 4 Kohlenstoffatome enthalten.

9. Mittel gemäß Anspruch 8, dadurch gekennzeichnet, daß $R_4$ für Halogenäthoxy steht.

10. Mittel gemäß Anspruch 8, dadurch gekennzeichnet, daß $R_4$ für Halogenmethoxy steht.

11. Mittel gemäß Anspruch 9, dadurch gekennzeichnet, daß $R_4$ den 2,2,2-Trifluoräthoxyrest bedeutet.

12. Mittel gemäß Anspruch 10, dadurch gekennzeichnet, daß $R_4$ den Difluormethoxyrest bedeutet.

13. Mittel gemäß Anspruch 1, dadurch gekennzeichnet, daß es als Wirkstoff N-(2-Difluormethoxyphenyl-sulfonyl)-N'-[4-methoxy-6-(2,2,2-trifluoräthoxy)-1,3,5-triazin-2-yl]-harnstoff enthält.

14. Mittel gemäß Anspruch 1, dadurch gekennzeichnet, daß es als Wirkstoff N-(2-Difluormethoxyphenyl-sulfonyl)-N'-(4-difluormethoxy-6-methyl-pyrimidin-2-yl)-harnstoff enthält.

15. Verfahren zur Herstellung der Verbindungen der Formel I, Anspruch 1, dadurch gekennzeichnet, daß man

a) ein Phenylsulfonamid der Formel II

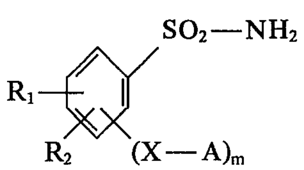

(II)

worin A, $R_1$, $R_2$, X und m die unter Formel I gegebene Bedeutung haben, in Gegenwart einer Base mit einem N-Pyrimidinyl- oder -Triazinylcarbamat der Formel

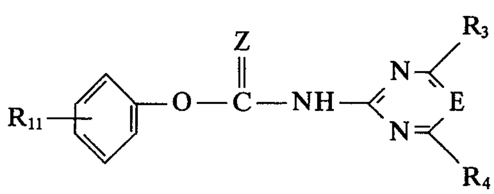

(III)

worin E, $R_3$, $R_4$ und Z die unter Formel I gegebene Bedeutung haben und $R_{11}$ für Wasserstoff, Halogen, Nitro oder $C_1$—$C_3$-Alkyl steht, umsetzt oder

b) ein Phenylsulfonylisocyanat oder -isothiocyanat der Formel IV

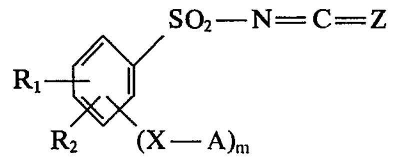

(IV)

worin A, $R_1$, $R_2$, m, X und Z die unter Formel I gegebene Bedeutung haben, gegebenenfalls in Gegenwart einer Base, mit einem Amin der Formel V

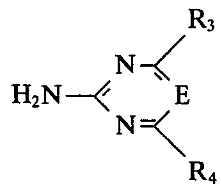

(V)

worin E, $R_3$ und $R_4$ die unter Formel I gegebene Bedeutung haben, umsetzt oder

c) Sulfonamide der oben angegebenen Formel II gegebenenfalls in Gegenwart einer Base mit einem Isocyanat oder Isothiocyanat der Formel VI

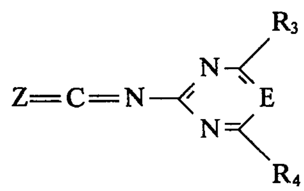

(VI)

worin E, $R_3$, $R_4$ und Z die unter Formel I gegebenen Bedeutungen haben, umsetzt oder

d) ein N-Phenylsulfonylcarbamat der Formel VII

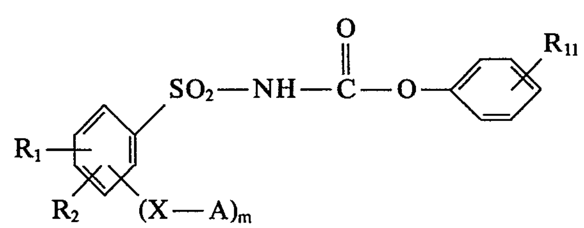

(VII)

worin A, $R_1$, $R_2$, m und X die unter Formel I gegebene Bedeutung haben und $R_{11}$ für Wasserstoff, Halogen, Nitro oder $C_1$—$C_3$-Alkyl steht, mit einem Amin der oben angegebenen Formel V umsetzt und gegebenenfalls in ihre Salze überführt, indem man einen Sulfonylharnstoff der Formel I mit einem Amin, einem Alkalimetall- oder Erdalkalimetallhydroxid oder einer quaternären Ammoniumbase umsetzt.

16. Die Verwendung der N-Phenylsulfonyl-N'-triazinyl- oder -pyrimidinyl-harnstoffe der Formel I oder sie enthaltender Mittel zur Bekämpfung unerwünschten Pflanzenwachstums.

17. Die Verwendung der N-Phenylsulfonyl-N'-triazinyl- oder -pyrimidinyl-harnstoffe der Formel I oder sie enthaltender Mittel zur Hemmung des Pflanzenwachstums.

18. Die Verwendung gemäß Anspruch 16 zur selektiven pre- oder postemergenten Bekämpfung von Unkräutern in Nutzpflanzenkulturen.

19. Die Verwendung gemäß Anspruch 18 in Getreide-, Mais-, Reis- und Baumwollkulturen.

20. Die Verwendung gemäß Anspruch 18 in Sojakulturen.

21. Die Verwendung gemäß Anspruch 17, zur Unterdrückung des Pflanzenwachstums über das 2-Blattstadium hinaus, dadurch gekennzeichnet, daß die Wirkstoffe preemergent angewendet werden.

22. Die Verwendung der N-Phenylsulfonyl-N'-triazinyl- oder -pyrimidinyl-harnstoffe der Formel I, Anspruch 1, oder sie enthaltender Mittel zur Wuchsregulierung von Kulturpflanzen zum Zwecke einer Ertragssteigerung.

23. Die Verwendung gemäß Anspruch 22 in Sojakulturen.

24. Die Verwendung gemäß Anspruch 17 bei Bodenbedecker-Leguminosen.

25. Mittel gemäß Anspruch 1, dadurch gekennzeichnet, daß es als Wirkstoff N-[2-(2-Chloräthoxy)-phenyl-sulfonyl]-N'-[4-methoxy-6-(2,2,2-trifluoräthoxy)-1,3,5-triazin-2-yl]-harnstoff enthält.

26. Mittel gemäß Anspruch 1, dadurch gekennzeichnet, daß es als Wirkstoff N-(2-Trifluormethoxy-phenyl-sulfonyl)-N'-(4-difluormethoxy-6-methyl-pyrimidin-2-yl)-harnstoff enthält.

**Claims for the Contracting States: DE, GB, FR, CH, LI, IT, NL, BE, SE**

1. An N-phenylsulfonyl-N'-pyrimidinylurea or N-phenylsulfonyl-N'-triazinylurea of the general formula I

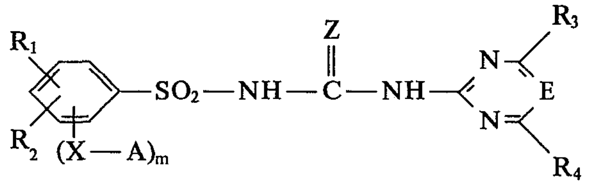

(I)

in which A is a $C_1$—$C_6$alkyl radical which is substituted by halogen, $C_1$—$C_4$alkoxy, $C_1$—$C_4$alkylthio, $C_1$—$C_4$alkylsulfinyl, $C_1$—$C_4$alkylsulfonyl, $C_1$—$C_4$haloalkoxy, $C_1$—$C_4$haloalkylthio, $C_1$—$C_4$haloalkylsulfinyl or $C_1$—$C_4$haloalkylsulfonyl, or is a $C_2$—$C_6$alkenyl radical substituted by the above radicals, E is the methine group or nitrogen, X is oxygen, sulfur or a sulfinyl or sulfonyl bridge, Z is oxygen or sulfur, m is one or two, $R_1$ is hydrogen, halogen, $C_1$—$C_5$alkyl, $C_2$—$C_5$alkenyl or a —Y—$R_5$ radical, $R_2$ is hydrogen, halogen, $C_1$—$C_5$alkyl, $C_2$—$C_5$alkenyl, $C_1$—$C_4$haloalkyl or a —Y—$R_5$, —$COOR_6$, —$NO_2$ or —CO—$NR_7$—$R_8$ radical, $R_3$ is hydrogen, $C_1$—$C_4$alkyl, $C_1$—$C_4$alkoxy, $C_1$—$C_4$alkylthio, $C_1$—$C_4$haloalkyl, halogen or $C_1$—$C_4$haloalkoxy, or alkoxyalkyl having not more than 4 carbon atoms, $R_4$ is $C_1$—$C_4$haloalkoxy, $R_5$ and $R_6$ are each $C_1$—$C_5$alkyl, $C_2$—$C_5$alkenyl or $C_2$—$C_6$alkynyl, $R_7$ and $R_8$ independently or each other are hydrogen, $C_1$—$C_5$alkyl, $C_2$—$C_5$alkenyl or $C_2$—$C_6$alkynyl and Y is oxygen, sulfur or a sulfinyl or sulfonyl bridge, with the proviso that A can also be an unstubstituted $C_2$—$C_6$alkenyl radical if X is simultaneously oxygen; or a salt thereof.

2. A compound according to claim 1, in which A is a $C_1$—$C_6$alkyl radical which is substituted by halogen, $C_1$—$C_4$alkoxy, $C_1$—$C_4$alkylthio, $C_1$—$C_4$alkylsulfinyl, $C_1$—$C_4$alkylsulfonyl, $C_1$—$C_4$haloalkoxy, $C_1$—$C_4$haloalkylthio, $C_1$—$C_4$haloalkylsulfinyl or $C_1$—$C_4$haloalkylsulfonyl, or is a $C_2$—$C_6$alkenyl radical substituted by the above radicals, E is the methine group or nitrogen, X is oxygen, sulfur or a sulfinyl or sulfonyl bridge, Z is oxygen or sulfur, m is one or two, $R_1$ is hydrogen, halogen, $C_1$—$C_5$alkyl, $C_2$—$C_5$alkenyl or a —Y—$R_5$ radical, $R_2$ is hydrogen, halogen, $C_1$—$C_5$alkyl, $C_2$—$C_5$alkenyl, $C_1$—$C_4$haloalkyl or a —Y—$R_5$, —$COOR_6$, —$NO_2$ or —CO—$NR_7$—$R_8$ radical, $R_3$ is hydrogen, $C_1$—$C_4$alkyl, $C_1$—$C_4$alkoxy, $C_1$—$C_4$alkylthio, $C_1$—$C_4$haloalkyl, halogen or alkoxyalkyl having not more than 4 carbon atoms, $R_4$ is $C_1$—$C_4$haloalkoxy, $R_5$ and $R_6$ are each $C_1$—$C_5$alkyl, $C_2$—$C_5$alkenyl or $C_2$—$C_6$alkynyl, $R_7$ and $R_8$ independently of each other are hydrogen, $C_1$— $C_5$alkyl, $C_2$—$C_5$alkenyl or $C_2$—$C_6$alkynyl and Y is oxygen, sulfur or a sulfinyl or sulfonyl bridge, with the proviso that A can also be an unstubstituted $C_2$—$C_6$alkenyl radical if X is simultaneously oxygen; or a salt thereof.

3. A compound according to claim 1, in which Z is oxygen.

4. A compound according to claim 1, in which X is oxygen.

5. A compound according to claim 1, in which the radicals $R_3$ and $R_4$ together contain not more than 4 carbon atoms.

6. A compound according to claim 1, in which m is one.

7. A compound according to claim 1, in which X and Z are oxygen, m is one and the radical —X—A is in the 2-position relative to the sulfonyl group.

8. A compound according to claim 7, in which the radicals $R_3$ and $R_4$ together contain not more than 4 carbon atoms.

9. A compound according to claim 8, in which $R_4$ is haloethoxy.

10. A compound according to claim 8, in which $R_4$ is halomethoxy.

11. A compound according to claim 9, in which $R_4$ is the 2,2,2-trifluoroethoxy radical.

12. A compound according to claim 10, in which $R_4$ is the difluoromethoxy radical.

13. N-(2-Difluoromethoxyphenylsulfonyl)-N'-[4-methoxy-6-(2,2,2-trifluoroethoxy)-1,3,5-triazin-2-yl]urea according to claim 1.

14. N-(2-Difluoromethoxyphenylsulfonyl)-N'-(4-difluoromethoxy-6-methylpyrimidin-2-yl)urea according to claim 1.

15. A process for the preparation of a compound of the formula I according to claim 1, which comprises reacting a phenylsulfonamide of the formula II

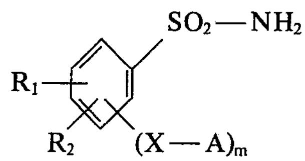

(II)

in which A, $R_1$, $R_2$, X and m are as defined for formula I, with an N-pyrimidinylcarbamate or N-triazinylcarbamate of the formula III

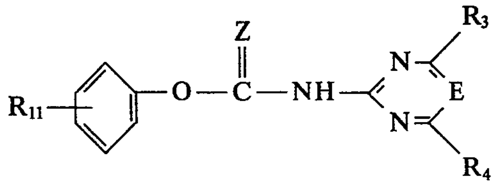

(III)

in which E, $R_3$, $R_4$ and Z are as defined for formula I and $R_{11}$ is hydrogen, halogen, nitro or $C_1$—$C_3$alkyl, in the presence of a base, and, if appropriate, converting the product into a salt thereof.

16. A process for the preparation of a compound of the formula I according to claim 1, which comprises reacting a phenylsulfonylisocyanate or phenylsulfonylisothiocyanate of the formula IV

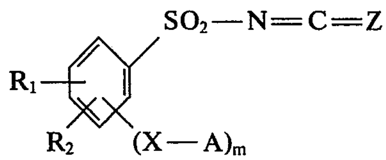

(IV)

in which A, $R_1$, $R_2$, m, X and Z are as defined for formula I, with an amine of the formula V

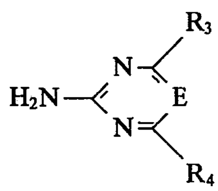

(V)

in which E, $R_3$ and $R_4$ are as defined for formula I, in the presence or absence of a base, and, if appropriate, converting the product into a salt thereof.

17. A process for the preparation of a compound of the formula I according to claim 1, which comprises reacting a sulfonamide of the above formula II with an isocyanate or isothiocyanate of the formula VI

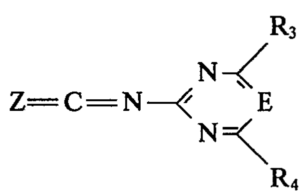

(VI)

in which E, $R_3$, $R_4$ and Z are as defined for formula I, in the presence or absence of a base, and, if appropriate, converting the product into a salt thereof.

18. A process for the preparation of a compound of the formula I according to claim 1, which comprises reacting an N-phenylsulfonylcarbamate of the formula VII

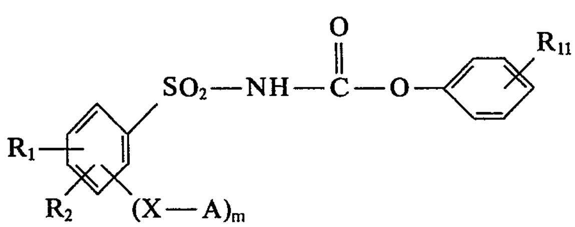

(VII)

in which A, $R_1$, $R_2$, m and X are as defined for formula I, and $R_{11}$ is hydrogen, halogen, nitro or $C_1$—$C_3$alkyl, with an amine or the formula V given above, and, if appropriate, converting the product into a salt thereof.

19. A process for the preparation of an addition salt of the formula I according to any one of claims 15 to 18, which comprises reacting a sulfonylurea of the formula I with an amine, an alkali metal hydroxide, an alkaline earth metal hydroxide or a quaternary ammonium base.

20. A herbicidal and plant growth inhibiting composition which, in addition to carriers and/or other adjuvants, contains as active ingredient at least one N-phenylsulfonyl-N'-triazinylurea or N-phenylsulfonyl-N'-pyrimidinylurea of the formula I according to claim 1.

21. Use of an N-phenylsulfonyl-N'-triazinylurea or N-phenylsulfonyl-N'-pyrimidinylurea of the formula I according to claim 1, or of a composition containing such a compound, for controlling undesired plant growth.
22. Use of an N-phenylsulfonyl-N'-triazinylurea or N-phenylsulfonyl-N'-pyrimidinylurea of the formula I according to claim 1, or of a composition containing such a compound, for inhibiting plant growth.
23. Use according to claim 21, for selectively controlling weeds in crops of useful plants preemergence or postemergence.
24. Use according to claim 23 in cereal, maize, rice and cotton crops.
25. Use according to claim 23 in soybean crops.
26. Use according to claim 22 for suppressing plant growth beyond the 2-leaf stage, which comprises applying the active ingredient preemergence.
27. Use of an N-phenylsulfonyl-N'-triazinylurea or N-phenylsulfonyl-N'-pyrimidinylurea of the formula I according to claim 1, or of a composition containing such a compound, for regulating the growth of crops for the purpose of increasing yield.
28. Use according to claim 27 in soybean crops.
29. Use according to claim 22 in leguminous cover crops.
30. N-[2-(2-Chloroethoxy)phenylsulfonyl]-N'-[4-methoxy-6-(2,2,2-trifluoroethoxy)-1,3,5-triazin-2-yl]urea according to claim 1.
31. N-(2-Trifluoromethoxyphenylsulfonyl)-N'-(4-difluoromethoxy-6-methylpyrimidin-2-yl)urea according to claim 1.

**Claims for the Contracting State: AT**

1. A herbicidal and plant growth inhibiting composition which, in addition to carriers and/or other adjuvants, contains as active ingredient at least one N-phenylsulfonyl-N'-triazinylurea or N-phenylsulfonyl-N'-pyrimidinylurea of the formula I

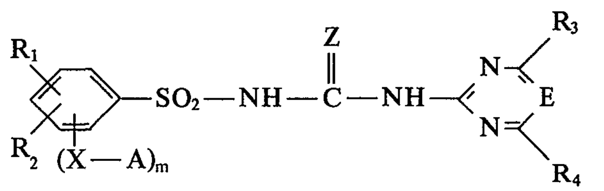

(I)

in which A is a $C_1$—$C_6$alkyl radical which is substituted by halogen, $C_1$—$C_4$alkoxy, $C_1$—$C_4$alkylthio, $C_1$—$C_4$alkylsulfinyl, $C_1$—$C_4$alkylsulfonyl, $C_1$—$C_4$haloalkoxy, $C_1$—$C_4$haloalkylthio, $C_1$—$C_4$haloalkylsulfinyl or $C_1$—$C_4$haloalkylsulfonyl, or is a $C_2$—$C_6$alkenyl radical substituted by the above radicals, E is the methine group or nitrogen, X is oxygen, sulfur or a sulfinyl or sulfonyl bridge, Z is oxygen or sulfur, m is one or two, $R_1$ is hydrogen, halogen, $C_1$—$C_5$alkyl, $C_2$—$C_5$alkenyl or a —Y—$R_5$ radical, $R_2$ is hydrogen, halogen, $C_1$—$C_5$alkyl, $C_2$—$C_5$alkenyl, $C_1$—$C_4$haloalkyl or a —Y—$R_5$, —$COOR_6$, —$NO_2$ or —CO—$NR_7$—$R_8$ radical, $R_3$ is hydrogen, $C_1$—$C_4$alkyl, $C_1$—$C_4$alkoxy, $C_1$—$C_4$alkylthio, $C_1$—$C_4$haloalkyl, halogen or $C_1$—$C_4$haloalkoxy, or alkoxyalkyl having not more than 4 carbon atoms, $R_4$ is $C_1$—$C_4$haloalkoxy, $R_5$ and $R_6$ are each $C_1$—$C_5$alkyl, $C_2$—$C_5$alkenyl or $C_2$—$C_6$alkynyl, $R_7$ and $R_8$ independently or each other are hydrogen, $C_1$—$C_5$alkyl, $C_2$—$C_5$alkenyl or $C_2$—$C_6$alkynyl and Y is oxygen, sulfur or a sulfinyl or sulfonyl bridge, with the proviso that A can also be an unstubstituted $C_2$—$C_6$alkenyl radical if X is simultaneously oxygen; or a salt thereof.
2. A composition according to claim 1, in which A is a $C_1$—$C_6$alkyl radical which is substituted by halogen, $C_1$—$C_4$alkoxy, $C_1$—$C_4$alkylthio, $C_1$—$C_4$alkylsulfinyl, $C_1$—$C_4$alkylsulfonyl, $C_1$—$C_4$haloalkoxy, $C_1$—$C_4$haloalkylthio, $C_1$—$C_4$haloalkylsulfinyl or $C_1$—$C_4$haloalkylsulfonyl, or is a $C_2$—$C_6$alkenyl radical substituted by the above radicals, E is the methine group or nitrogen, X is oxygen, sulfur or a sulfinyl or sulfonyl bridge, Z is oxygen or sulfur, m is one or two, $R_1$ is hydrogen, halogen, $C_1$—$C_5$alkyl, $C_2$—$C_5$alkenyl or a —Y—$R_5$ radical, $R_2$ is hydrogen, halogen, $C_1$—$C_5$alkyl, $C_2$—$C_5$alkenyl, $C_1$—$C_4$haloalkyl or a —Y—$R_5$, —$COOR_6$, —$NO_2$ or —CO—$NR_7$—$R_8$ radical, $R_3$ is hydrogen, $C_1$—$C_4$alkyl, $C_1$—$C_4$alkoxy, $C_1$—$C_4$alkylthio, $C_1$—$C_4$haloalkyl, halogen or alkoxyalkyl having not more than 4 carbon atoms, $R_4$ is $C_1$—$C_4$haloalkoxy, $R_5$ and $R_6$ are each $C_1$—$C_5$alkyl, $C_2$—$C_5$alkenyl or $C_2$—$C_6$alkynyl, $R_7$ and $R_8$ independently of each other are hydrogen, $C_1$—$C_5$alkyl, $C_2$—$C_5$alkenyl or $C_2$—$C_6$alkynyl and Y is oxygen, sulfur or a sulfinyl or sulfonyl bridge, with the proviso that A can also be an unstubstituted $C_2$—$C_6$alkenyl radical if X is simultaneously oxygen; or a salt thereof.
3. A composition according to claim 1, in which Z is oxygen.
4. A composition according to claim 1, in which X is oxygen.
5. A composition according to claim 1, in which the radicals $R_3$ and $R_4$ together contain not more than 4 carbon atoms.
6. A composition according to claim 1, in which m is one.

7. A composition according to claim 1, in which X and Z are oxygen, m is one and the radical —X—A is in the 2-position relative to the sulfonyl group.

8. A composition according to claim 1, in which the radicals $R_3$ and $R_4$ together contain not more than 4 carbon atoms.

9. A composition according to claim 8, in which $R_4$ is haloethoxy.

10. A composition according to claim 8, in which $R_4$ is halomethoxy.

11. A composition according to claim 9, in which $R_4$ is the 2,2,2-trifluoroethoxy radical.

12. A composition according to claim 10, in which $R_4$ is the difluoromethoxy radical.

13. A composition according to claim 1, which contains as active ingredient N-(2-difluoromethoxyphenylsulfonyl)-N'-[4-methoxy-6-(2,2,2-trifluoroethoxy)-1,3,5-triazin-2-yl]urea.

14. A composition according to claim 1, which contains as active ingredient N-(2-difluoromethoxyphenylsulfonyl)-N'-[4-methoxy-6-methylpyrimidin-2-yl]urea.

15. A process for the preparation of a compound of the formula I, which comprises

a) reacting a phenylsulfonamide of the formula II

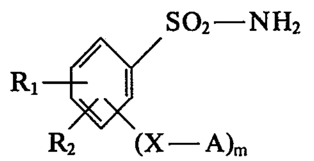

(II)

in which A, $R_1$, $R_2$, X and m are as defined for formula I, with an N-pyrimidinylcarbamate or N-triazinylcarbamate of the formula III

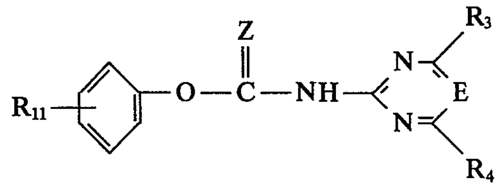

(III)

in which E, $R_3$, $R_4$ and Z are as defined for formula I and $R_{11}$ is hydrogen, halogen, nitro or $C_1$—$C_3$alkyl, in the presence of a base, or

b) reacting a phenylsulfonylisocyanate or phenylsulfonylisothiocyanate of the formula IV

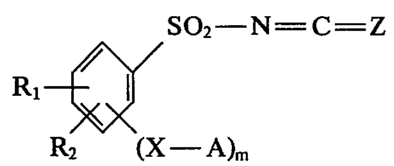

(IV)

in which A, $R_1$, $R_2$, m, X and Z are as defined for formula I, with an amine of the formula V

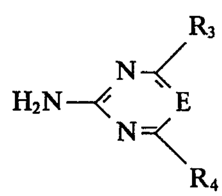

(V)

in which E, $R_3$ and $R_4$ are as defined for formula I, in the presence or absence of a base, or

c) reacting a sulfonamide of the above formula II with an isocyanate or isothiocyanate of the formula VI

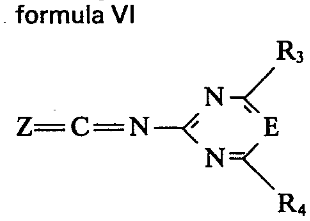

(VI)

in which E, $R_3$, $R_4$ and Z are as defined for formula I, in the presence or absence of a base, or

d) reacting an N-phenylsulfonylcarbamate of the formula VII

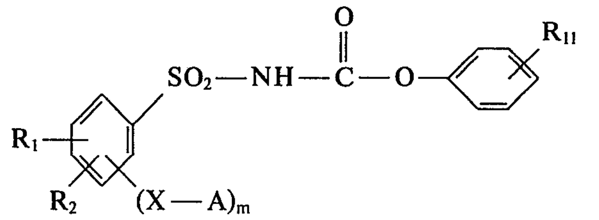

(VII)

in which A, $R_1$, $R_2$, m and X are as defined for formula I, and $R_{11}$ is hydrogen, halogen, nitro or $C_1$—$C_3$alkyl, with an amine of the formula V given above, and, if appropriate, converting the resultant sulfonylurea of formula I into a salt thereof by reaction with an amine, an alkali metal hydroxide, an alkaline earth metal hydroxide or a quaternary ammonium base.

16. Use of an N-phenylsulfonyl-N'-triazinylurea or N-phenylsulfonyl-N'-pyrimidinylurea of the formula I according to claim 1, or of a composition containing such a compound, for controlling undesired plant growth.

17. Use of an N-phenylsulfonyl-N'-triazinylurea or N-phenylsulfonyl-N'-pyrimidinylurea of the formula I according to claim 1, or of a composition containing such a compound, for inhibiting plant growth.

18. Use according to claim 16, for selectively controlling weeds in crops of useful plants preemergence or postemergence.

19. Use according to claim 18 in cereal, maize, rice and cotton crops.

20. Use according to claim 18 in soybean crops.

21. Use according to claim 17 for suppressing plant growth beyond the 2-leaf stage, which comprises applying the active ingredient preemergence.

22. Use of an N-phenylsulfonyl-N'-triazinylurea or N-phenylsulfonyl-N'-pyrimidinylurea of the formula I according to claim 1, or of a composition containing such a compound, for regulating the growth of crops for the purpose of increasing yield.

23. Use according to claim 22 in soybean crops.

24. Use according to claim 17 in leguminous cover crops.

25. A composition according to claim 1, which contains as active ingredient N-[2-(2-chloroethoxy)-phenylsulfonyl]-N'-[4-methoxy-6-(2,2,2-trifluoroethoxy)-1,3,5-triazin-2-yl]urea.

26. A composition according to claim 1, which contains as active ingredient N-(2-trifluoromethoxy-phenylsulfonyl)-N'-(4-difluoromethoxy-6-methylpyrimidin-2-yl)urea.

**Revendications pour les Etats contractants: DE, GB, FR, CH, LI, IT, NL, BE, SE**

1. N-phénylsulfonyl-N'-pyrimidinyl- et triazinyl-urées de formule générale I

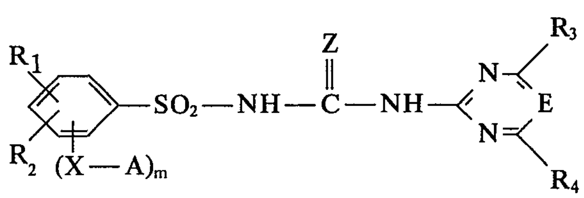

(I)

dans laquelle

A est un reste alkyle $C_1$—$C_6$ substitué par un halogène, alcoxy $C_1$—$C_4$, alkylthio $C_1$—$C_4$, alkylsulfinyle $C_1$—$C_4$, alkylsulfonyle $C_1$—$C_4$, halogénoalcoxy $C_1$—$C_4$, halogénoalkylthio $C_1$—$C_4$, halogénoalkylsulfinyle $C_1$—$C_4$, ou halogénoalkylsulfonyle ou bien un reste alcényle $C_2$—$C_6$ substitué par les restes énumérés,

E est le groupe méthine ou l'azote,

X est l'oxygène, le soufre, un pont sulfinyle ou sulfonyle,

Z est l'oxygène ou le soufre,

m est le nombre un ou deux,

$R_1$ est un hydrogène, halogène, alkyle $C_1$—$C_5$, alcényle $C_2$—$C_5$ ou un reste —Y—$R_5$;

$R_2$ est un hydrogène, halogène, alkyle $C_1$—$C_5$, alcényle $C_2$—$C_5$, halogénoalkyle $C_1$—$C_4$, ou un reste —Y—$R_5$, —$COOR_6$, —$NO_2$ ou —CO—$NR_7$—$R_8$,

$R_3$ est un hydrogène, alkyle $C_1$—$C_4$, alcoxy $C_1$—$C_4$, alkylthio $C_1$—$C_4$, halogénoalkyle $C_1$—$C_4$, halogène, halogénoalcoxy $C_1$—$C_4$ ou alcoxyalkyle avec au plus 4 atomes de carbone,

$R_4$ est un halogénoalcoxy $C_1$—$C_4$,

$R_5$ et $R_6$ sont chacun un alkyle $C_1$—$C_5$, alcényle $C_2$—$C_5$ ou alcynyle $C_2$—$C_6$,
$R_7$ et $R_8$ sont indépendament l'un de l'autre un hydrogène, alkyle $C_1$—$C_5$, alcényle $C_2$—$C_5$ ou alcynyle $C_2$—$C_6$ et
Y est l'oxygène, soufre, un pont sulfinyle ou sulfonyle,

dans laquelle A peut être aussi le reste alcényle $C_2$—$C_6$ non-substitué quand, simultanément X est l'oxygène, ainsi que les sels de ces composés.

2. Composés selon la revendication 1, caractérisé en ce que

A est un reste alkyle $C_1$—$C_6$ substitué par un halogène, alcoxy $C_1$—$C_4$, alkylthio $C_1$—$C_4$, alkylsulfinyle $C_1$—$C_4$, alkylsulfonyle $C_1$—$C_4$, halogénoalcoxy $C_1$—$C_4$, halogénoalkylthio $C_1$—$C_4$, halogénoalkylsulfinyle $C_1$—$C_4$ ou halogénoalkylsulfonyle ou bien un reste alcényle $C_2$—$C_6$ substitué par les restes énumérés,
E est le groupe méthine ou l'azote,
X est l'oxygène, le soufre, un pont sulfinyle ou sulfonyle,
Z est l'oxygène ou le soufre,
m est le nombre un ou deux,
$R_1$ est un hydrogène, halogène, alkyle $C_1$—$C_5$, alcényle $C_2$—$C_5$ ou un reste $-Y-R_5$;
$R_2$ est un hydrogène, halogène, alkyle $C_1$—$C_5$, alcényle $C_2$—$C_5$, halogénoalkyle $C_1$—$C_4$, ou un reste $-Y-R_5$, $-COOR_6$, $-NO_2$ ou $-CO-NR_7-R_8$,
$R_3$ est un hydrogène, alkyle $C_1$—$C_4$, alcoxy $C_1$—$C_4$, alkylthio $C_1$—$C_4$, halogénoalkyle $C_1$—$C_4$, halogène, halogénoalcoxy $C_1$—$C_4$ ou alcoxyalkyle avec au plus 4 atomes de carbone,
$R_4$ est un halogénoalcoxy $C_1$—$C_4$,
$R_5$ et $R_6$ sont chacun un alkyle $C_1$—$C_5$, alcényle $C_2$—$C_5$ ou alcynyle $C_2$—$C_6$,
$R_7$ et $R_8$ sont indépendament l'un de l'autre un hydrogène, alkyle $C_1$—$C_5$, alcényle $C_2$—$C_5$ ou alcynyle $C_2$—$C_6$ et
Y est l'oxygène, soufre, un pont sulfinyle ou sulfonyle,

dans laquelle A peut être aussi le reste alcényle $C_2$—$C_6$ non-substitué quand, simultanément X est l'oxygène, ainsi que les sels de ces composés.

3. Composés selon la revendication 1 caractérisés en ce que Z est l'oxygène.

4. Composés selon la revendication 1 caractérisés en ce que X est l'oxygène.

5. Composés selon la revendication 1 caractérisés en ce que les restes $R_3$ et $R_4$ ensemble ne contiennent pas plus de 4 atomes de carbone.

6. Composés selon la revendication 1 caractérisés en ce que m est le nombre un.

7. Composés selon la revendication 1 caractérisés en ce que X et Z sont l'oxygène et m est un et que le reste $-X-A$ est en position 2 par rapport au groupe sulfonyle.

8. Composés selon la revendication 7 caractérisés en ce que les restes $R_3$ et $R_4$ ensemble ne contiennent pas plus de 4 atomes de carbone.

9. Composés selon la revendication 8 caractérisés en ce que $R_4$ est un halogénoéthoxy.

10. Composés selon la revendication 8 caractérisés en ce que $R_4$ est un halogénométhoxy.

11. Composés selon la revendication 9 caractérisés en ce que $R_4$ est le reste 2,2,2-trifluoroéthoxy.

12. Composés selon la revendication 10 caractérisés en ce que $R_4$ est le reste difluorométhoxy.

13. N-(2-difluorométhoxyphényl-sulfonyl)-N'-[4-méthoxy-6-(2,2,2-trifluoroéthoxy)-1,3,5-triazine-2-yl]-urée selon la revendication 1.

14. N-(2-difluorométhoxyphényl-sulfonyl)-N'-(4-difluorométhoxy-6-méthyl-pyrimidine-2-yl)urée selon la revendication 1.

15. Procédé de préparation de composés de formule I selon la revendication 1, caractérisé en ce qu'on additionne un phénylsulfonamide de formule II

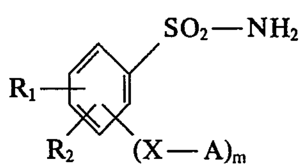

(II)

dans laquelle A, $R_1$, $R_2$, X et m ont la signification donnée pour la formule I en présence d'une base avec un N-pyrimidinyl- ou -triazinylcarbamate de formule III

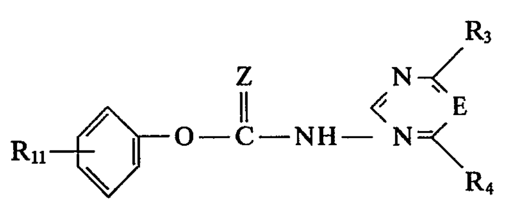

(III)

dans laquelle E, $R_3$, $R_4$ et Z ont la signification donnée pour la formule I et $R_{11}$ est hydrogène, halogène, nitro ou alkyle $C_1—C_3$, et transforme le cas échéant en leurs sels.

16. Procédé de préparation de composés de formule I selon la revendication 1 caractérisé en ce qu'on additionne un phényl sulfonylisocyanate ou -isothiocyanate de formule IV

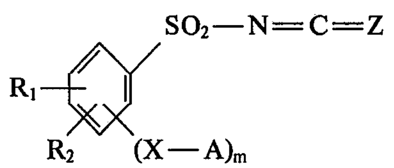

(IV)

dans laquelle A, $R_1$, $R_2$, m, X et Z ont la signification donnée pour la formule I, le cas échéant en présence d'une base avec une amine de formule V

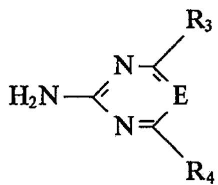

(V)

dans laquelle E, $R_3$ et $R_4$ ont la signification donnée pour la formule I et transforme le cas échéant en leurs sels.

17. Procédé de préparation de composés de formule I selon la revendication 1 caractérisé en ce qu'on additionne des sulfonamides de la formule II donnée ci-dessus le cas échéant en présence d'une base avec un isocyanate ou isothiocyanate de formule VI

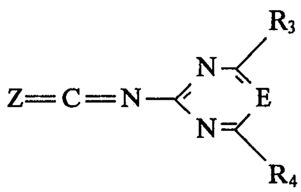

(VI)

dans laquelle E, $R_3$, $R_4$ et Z ont les significations données pour la formule I et transforme le cas échéant en leurs sels.

18. Procédés de préparation de composés de formule I selon la revendication 1 caractérisé en ce qu'on additionne un N-phénylsulfonylcarbamate de formule VII

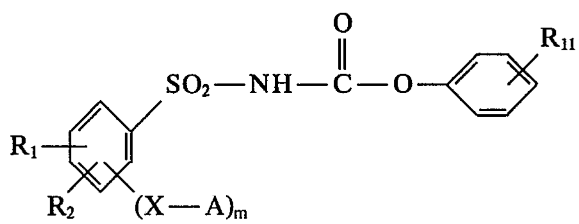

(VII)

dans laquelle A, $R_1$, $R_2$, m et X ont la signification donnée pour la formule I et $R_{11}$ est hydrogène, halogène, nitro ou alkyle $C_1—C_3$ avec une amine de formule V donnée ci-dessus et transforme le cas échéant en leurs sels.

19. Procédé de préparation de sels d'addition de formule I selon l'une des revendications 15 à 18 caractérisé en ce qu'on additionne une sulfonylurée de formule I avec une amine, un hydroxyde de métal alcalin ou de métal alcalinoterreux ou une base ammonium quaternaire.

20. Moyen herbicide ou empêchant la croissance des plantes caractérisé en ce qu'il contient, à côté de produits support et/ou autres additifs comme produit actif au moins une N-phényl-sulfonyl-N'-triazinyl- ou -pyrimidinyl-urée de formule I selon la revendication 1.

21. Utilisation de N-phénylsulfonyl-N'-triazinyl- ou -pyrimidinyl-urée de formule I, selon la revendication 1, ou des moyens les renfermant pour combattre la croissance indésirable de plantes.

22. Utilisation de N-phénylsulfonyl-N'-triazinyl- ou -pyrimidinylurée de formule 1, selon la revendication 1, ou de moyens les renfermant pour empêcher la croissance des plantes.

23. Utilisation selon la revendication 21 pour combattre sélectivement les mauvaises herbes avant ou après la levée dans les cultures de plantes utiles.

24. Utilisation selon la revendication 23 dans les cultures de céréales, maïs, riz et coton.

25. Utilisation selon la revendication 24 dans les cultures de soja.

26. Utilisation selon la revendication 22 pour empêcher la croissance de plantes au delà de la 2ème pousée de feuille, caractérisée en ce que les produits actifs sont utilisés avant la levée.

27. Utilisation de N-phénylsulfonyl-N'-triazinyl- ou -pyrimidinyl-urées de formule I, selon la revendication 1, ou de moyens les renfermant pour régulariser la croissance de plantes cultivées dans le but d'augmenter le rendement.

28. Utilisation selon la revendication 27 dans les cultures de soja.

29. Utilisation selon la revendication 22 pour les légumineuses de couverture de sol.

30. N-[2-(2-chloroéthoxy)-phényl-sulfonyl]-N'-[4-méthoxy-6-(2,2,2-trifluoroéthoxy)-1,3,5-triazin-2-yl]-urée, selon la revendication 1.

31. N-(2-trifluorométhoxyphényl-sulfonyl)-N'-(4-difluorométhoxy-6-méthyl-pyrimidin-2-yl)-urée selon la revendication 1.

**Revendications pour l'Etat contractant: AT**

1. Agent herbicide et empêchant la croissance de plantes caractérisé en ce qu'à côte de supports et autres additifs il contienne au moins une N-phénylsulfonyl-N'-pyrimidinyl- et triazinyl-urées de formule générale I

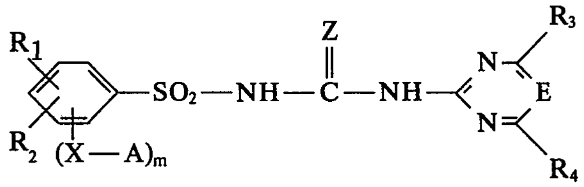

(I)

ou un de ses sels
dans laquelle

- A est un reste alkyle $C_1$—$C_6$ substitué par un halogène, alcoxy $C_1$—$C_4$, alkylthio $C_1$—$C_4$, alkylsulfinyle $C_1$—$C_4$, alkylsulfonyle $C_1$—$C_4$, halogénoalcoxy $C_1$—$C_4$, halogénoalkylthio $C_1$—$C_4$, halogénoalkylsulfinyle $C_1$—$C_4$, ou halogénoalkylsulfonyle ou bien un reste alcényle $C_2$—$C_6$ substitué par les restes énumérés,
- E est le groupe méthine ou l'azote,
- X est l'oxygène, le soufre, un pont sulfinyle ou sulfonyle,
- Z est l'oxygène ou le soufre,
- m est le nombre un ou deux,
- $R_1$ est un hydrogène, halogène, alkyle $C_1$—$C_5$, alcényle $C_2$—$C_5$ ou un reste —Y—$R_5$
- $R_2$ est un hydrogène, halogène, alkyle $C_1$—$C_5$, alcényle $C_2$—$C_5$, halogénoalkyle $C_1$—$C_4$, ou un reste —Y—$R_5$, —$COOR_6$, —$NO_2$ ou —CO—$NR_7$—$R_8$,
- $R_3$ est un hydrogène, alkyle $C_1$—$C_4$, alcoxy $C_1$—$C_4$, alkylthio $C_1$—$C_4$, halogénoalkyle $C_1$—$C_4$, halogène, halogénoalcoxy $C_1$—$C_4$ ou alcoxyalkyle avec au plus 4 atomes de carbone,
- $R_4$ est un halogénoalcoxy $C_1$—$C_4$,
- $R_5$ et $R_6$ sont chacun un alkyle $C_1$—$C_5$, alcényle $C_2$—$C_5$ ou alcynyle $C_2$—$C_6$,
- $R_7$ et $R_8$ sont indépendament l'un de l'autre un hydrogène, alkyle $C_1$—$C_5$, alcényle $C_2$—$C_5$ ou alcynyle $C_2$—$C_6$ et
- Y est l'oxygène, soufre, un pont sulfinyle ou sulfonyle,

dans laquelle A peut être aussi le reste alcényle $C_2$—$C_6$ non-substitué quand, simultanément X est l'oxygène.

2. Moyen selon la revendication 1 caractérisé en ce que

- A est un reste alkyle $C_1$—$C_6$ substitué par un halogène, alcoxy $C_1$—$C_4$, alkylthio $C_1$—$C_4$, alkylsulfinyle $C_1$—$C_4$, alkylsulfonyle $C_1$—$C_4$, halogénoalcoxy $C_1$—$C_4$, halogénoalkylthio $C_1$—$C_4$, halogénoalkylsulfinyle $C_1$—$C_4$ ou halogénoalkylsulfonyle ou bien un reste alcényle $C_2$—$C_6$ substitué par les restes énumérés,
- E est le groupe méthine ou l'azote,
- X est l'oxygène, le soufre, un pont sulfinyle ou sulfonyle,
- Z est l'oxygène ou le soufre,
- m est le nombre un ou deux,
- $R_1$ est un hydrogène, halogène, alkyle $C_1$—$C_5$, alcényle $C_2$—$C_5$ ou un reste —Y—$R_5$
- $R_2$ est un hydrogène, halogène, alkyle $C_1$—$C_5$, alcényle $C_2$—$C_5$, halogénoalkyle $C_1$—$C_4$, ou un reste —Y—$R_5$, —$COOR_6$, —$NO_2$ ou —CO—$NR_7$—$R_8$,
- $R_3$ est un hydrogène, alkyle $C_1$—$C_4$, alcoxy $C_1$—$C_4$, alkylthio $C_1$—$C_4$, halogénoalkyle $C_1$—$C_4$, halogène, halogénoalcoxy $C_1$—$C_4$, ou alcoxyalkyle avec au plus 4 atomes de carbone,

$R_4$ est un halogénoalcoxy $C_1$—$C_4$,
$R_5$ et $R_6$ sont chacun un alkyle $C_1$—$C_5$, alcényle $C_2$—$C_5$ ou alcynyle $C_2$—$C_6$,
$R_7$ et $R_8$ sont indépendament l'un de l'autre un hydrogène, alkyle $C_1$—$C_5$, alcényle $C_2$—$C_5$ ou alcynyle $C_2$—$C_6$ et
Y est l'oxygène, soufre, un pont sulfinyle ou sulfonyle,

dans laquelle A peut être aussi le reste alcényle $C_2$—$C_6$ non-substitué quand, simultanément X est l'oxygène.

3. Moyen selon la revendication 1 caractérisé en ce que Z est l'oxygène.
4. Moyen selon la revendication 1 caractérisé en ce que X est l'oxygène.
5. Moyen selon la revendication 1 caractérisé en ce que les restes $R_3$ et $R_4$ ensemble ne contiennent pas plus de 4 atomes de carbone.
6. Moyen selon la revendication 1 caractérisé en ce que m est le nombre un.
7. Moyen selon la revendication 1 caractérisé en ce que X et Z sont l'oxygène et m est un et que le reste —X—A est en position 2 par rapport au groupe sulfonyle.
8. Moyen selon la revendication 7 caractérisé en ce que les restes $R_3$ et $R_4$ ensemble ne contiennent pas plus de 4 atomes de carbone.
9. Moyen selon la revendication 8 caractérisé en ce que $R_4$ est un halogénoéthoxy.
10. Moyen selon la revendication 8 caractérisé en ce que $R_4$ est un halogénométhoxy.
11. Moyen selon la revendication 9 caractérisé en ce que $R_4$ est le reste 2,2,2-trifluoroéthoxy.
12. Moyen selon la revendication 10 caractérisé en ce que $R_4$ est le reste difluorométhoxy.
13. Moyen selon la revendication 1 caractérisé en ce qu'il contienne comme produit actif de la N-(2-difluorométhoxyphényl-sulfonyl)-N'-[4-méthoxy-6-(2,2,2-trifluoroéthoxy)-1,3,5-triazine-2-yl]-urée
14. Moyen selon la revendication 1 caractérisé en ce qu'il contienne comme produit actif de la N-(2-difluorométhoxyphényl-sulfonyl)-N'-(4-difluorométhoxy-6-méthyl-pyrimidine-2-yl)urée.
15. Procédé de préparation de composés de formule I selon la revendication 1, caractérisé en ce qu'on additionne

a) un phénylsulfonamide de formule II

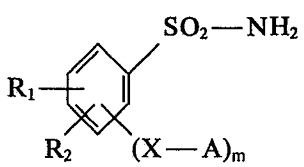

(II)

dans laquelle A, $R_1$, $R_2$, X et m ont la signification donnée pour la formule I en présence d'une base avec un N-pyrimidinyl- ou -triazinylcarbamate de formule

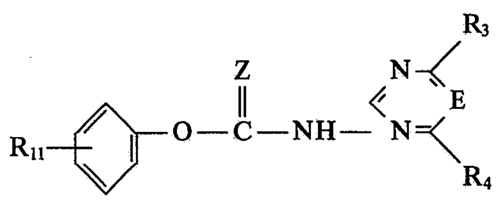

(III)

dans laquelle E, $R_3$, $R_4$ et Z ont la signification donnée pour la formule I et $R_{11}$ est hydrogène, halogène, nitro ou alkyle $C_1$—$C_3$ ou

b) un phényl sulfonylisocyanate ou -isothiocyanate de formule IV

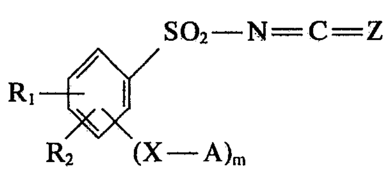

(IV)

dans laquelle A, $R_1$, $R_2$, m, X et Z ont la signification donnée pour la formule I, le cas échéant en présence d'une base avec une amine de formule V

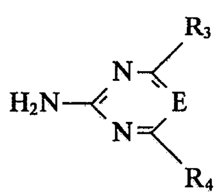

(V)

dans laquelle E, $R_3$ et $R_4$ ont la signification donnée pour la formule I ou

c) des sulfonamides de la formule II donnée cidessus le cas échéant en présence d'une base avec un isocyanate ou isothiocyanate de formule VI

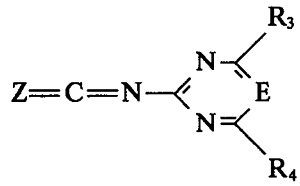

(VI)

dans laquelle E, $R_3$, $R_4$ et Z ont les significations données pour la formule I ou

d) un N phénylsulfonylcarbamate de formule VII

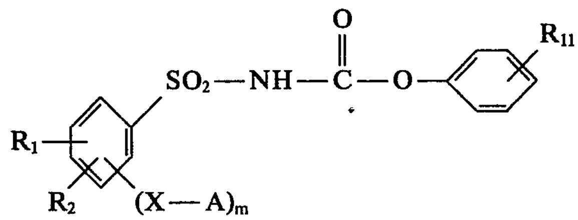

(VII)

dans laquelle A, $R_1$, $R_2$, m et X ont la signification donnée pour la formule I et $R_{11}$ est hydrogène, halogène, nitro ou alkyle $C_1$—$C_3$ avec une amine de formule V donnée ci-dessus et transforme le cas échéant en leurs sels, en additionnant une sulfonylurée de formule I avec une amine, un hydroxyde de métal alcalin ou de métal alcalinoterreux ou une base ammonium quaternaire.

16. Utilisation de N-phénylsulfonyl-N'-triazinyl- ou -pyrimidinyl-urée de formule I ou des moyens les renfermant pour combattre la croissance de plantes indésirables.

17. Utilisation de la N-phénylsulfonyl-N'-triazinyl- ou -pyrimidinyl-urée de formule I ou des moyens les renfermant pour empêcher la croissance des plantes.

18. Utilisation selon la revendication 16 pour combattre sélectivement les mauvaises herbes avant ou après la levée dans les cultures de plantes utiles.

19. Utilisation selon la revendication 18 dans les cultures de céréales, maïs, riz et coton.

20. Utilisation selon la revendication 18 dans les cultures de soja.

21. Utilisation selon la revendication 17 pour empêcher la croissance de plantes au delà de la 2éme pousée de feuille, caractérisée en ce que les produits actifs sont utilisés avant la levée.

22. Utilisation de N-phénylsulfonyl-N'-triazinyl- ou -pyrimidinyl-urées de formule I, selon la revendication 1, ou des moyens les renfermant pour régulariser la croissance de plantes cultivées dans le but d'augmenter le rendement.

23. Utilisation selon la revendication 22 dans les cultures de soja.

24. Utilisation selon la revendication 17 pour les légumineuses de couverture de sol.

25. Moyen selon la revendication 1 caractérisé en ce qu'il contient comme produit actif de la N-[2-(2-chloroéthoxy)-phényl-sulfonyl]-N'-[4-méthoxy-6-(2,2,2-trifluoroéthoxy)-1,3,5-triazine-2-yl]-urée.

26. Moyen selon la revendication 1, caractérisé en ce qu'il contient comme produit actif de la N-(2-trifluorométhoxyphényl-sulfonyl)-N'-(4-difluorométhoxy-6-méthyl-pyrimidin-2-yl)-urée.